# EUROPEAN PATENT APPLICATION

(11) **EP 4 640 628 A1**
(43) Date of publication of application: **29.10.2025**
(21) Application number: 23906513.9
(22) Date of filing: 09.11.2023
(51) Int. Cl.: C01B 32/80, C07C 68/02, C07C 69/96, C07C 263/10, C07C 265/04

(54) **METHOD FOR PRODUCING CARBONYL HALIDE**

(30) Priority: 21.12.2022 JP 2022203823
(71) Applicant: National University Corporation Kobe University, Kobe-shi, Hyogo 657-8501 (JP); MITSUBISHI GAS CHEMICAL COMPANY, INC., Chiyoda-ku Tokyo 100-8324 (JP)
(72) Inventor: TSUDA, Akihiko, Kobe-shi, Hyogo 657-8501 (JP)
(74) Representative: Müller-Boré & Partner Patentanwälte PartG mbB
(86) International application number: PCT/JP2023/040345
(87) International publication number: WO 2024/135142

(57) **Abstract**

The objective of the present invention is to provide a method for producing a carbonyl halide safely and efficiently. The method for producing a carbonyl halide according to the present invention is characterized in comprising the step of irradiating a light to a halomethane having 1 or more halogeno groups selected from the group consisting of chloro, bromo and iodo in the presence of oxygen and ozone.

## Description

### TECHNICAL FIELD

The present invention relates to a method for producing a carbonyl halide safely and efficiently.

### BACKGROUND ART

A carbonyl halide such as phosgene is very important as a synthetic intermediate of various compounds and a raw material of various materials. For example, a carbonate compound is generally produced from phosgene and an alcohol compound.

Phosgene is however very toxic. For example, phosgene easily reacts with water to generate hydrogen chloride and has a history of being used as poisonous gas. Phosgene is generally produced by a high-heat-generating gas-phase reaction between anhydrous chlorine gas and highly pure carbon monoxide in the presence of an activated carbon catalyst. Carbon monoxide used in this reaction is also toxic. The basic process to produce phosgene has not significantly changed since the 1920s. The production of phosgene by such a process requires expensive and huge facilities. In addition, it is essential for plant design to ensure a wide range of safety due to high toxicity of phosgene. Thus, the production cost increases.

The research group of the inventor of the present invention accordingly has developed a method for producing a halogen and/or a carbonyl halide by irradiating a light to a halogenated hydrocarbon such as chloroform in the presence of oxygen (Patent document 1). In addition, the research group found that a carbonyl halide can be efficiently produced by irradiating a high energy light to a mixed gas of a C₂₋₄ halogenated hydrocarbon and oxygen in a gas phase (Patent document 2). Furthermore, the research group also found that a carbonyl halide can be efficiently produced by irradiating a light to a composition containing a C₁₋₄ halogenated hydrocarbon in the presence of oxygen and a substance to generate a radical by visible light, such as chlorine (Patent document 3).

It is described in Non-patent document 1 that a chlorine atom initiates a reaction to oxidize tetrachloroethylene (Cl₂C=CCl₂) and thus phosgene or the like is produced. Non-patent document 1 also discloses that ozone is used.

### PRIOR ART DOCUMENT

### PATENT DOCUMENT

Patent document 1: JP 2013-181028 A
Patent document 2: WO 2022/172745
Patent document 3: WO 2021/045105

### NON-PATENT DOCUMENT

Non-patent document 1: ECKART MATHIAS, et al., Can. J. Chem., VOL. 52, 1974, 3852-3862

### DISCLOSURE OF THE INVENTION

### PROBLEMS TO BE SOLVED BY THE INVENTION

It has been known that chlorine accelerates an oxidative photolytic degradation of a carbonyl halide as described above.

But since chlorine is highly corrosive and toxic, a corrosive-resistant specially equipped facility and the introduction of a safety facility are required to treat chlorine. As a result, the production cost is increased. Under such a situation, an additive that is safer than chlorine, less toxic nor corrosive and can be used at lower cost has been required.

Thus, the objective of the present invention is to provide a method for producing a carbonyl halide safely and efficiently.

### MEANS FOR SOLVING THE PROBLEMS

The inventor of the present invention has made extensive studies to solve the above-described problems. As a result, the inventor completed the present invention by finding that a carbonyl halide can be produced safely and efficiently without using a halogen such as chlorine by oxidatively photolyzing a halomethane with using ozone and oxygen in combination, though Non-Patent Document 1 concludes that ozone inhibits the oxidation reaction of tetrachloroethylene initiated by a chlorine atom.

Hereinafter, the present invention is described.
[1] A method for producing a carbonyl halide, comprising the step of irradiating a light to a halomethane having 1 or more halogeno groups selected from the group consisting of chloro, bromo and iodo in the presence of oxygen and ozone.
[2] The method according to the above [1], wherein the light is irradiated to a mixed gas comprising the halomethane, the oxygen and the ozone.
[3] The method according to the above [1] or [2], wherein a ratio of the ozone to a total of the oxygen and the ozone is 1 vol% or more and 20 vol% or less.
[4] The method according to the above [2], wherein a volume ratio of the halomethane to the ozone is 0.1 times or more and 50 times or less.
[5] The method according to the above [2] or [4], wherein the mixed gas does not comprise chlorine.
[6] The method according to any one of the above [1] to [5], wherein the light is irradiated to the halomethane for 60 seconds or more and 5000 seconds or less.
[7] The method according to any one of the above [1] to [6], wherein the light is irradiated to the halomethane at a temperature of 50°C or higher and 200°C or lower.
[8] A method for producing a carbonate compound, the method comprising the steps of:
   producing a carbonyl halide by the method according to any one of the above [1] to [7], and
   reacting an alcohol compound and the carbonyl halide.
[9] A method for producing a halogenated formate ester compound, the method comprising the steps of:
   producing a carbonyl halide by the method according to any one of the above [1] to [7], and
   reacting an alcohol compound and the carbonyl halide.
[10] A method for producing an isocyanate compound, the method comprising the steps of:
   producing a carbonyl halide by the method according to any one of the above [1] to [7], and
   reacting a primary amine compound and the carbonyl halide.
[11] A method for producing a carbamoyl halide compound, the method comprising the steps of:
   producing a carbonyl halide by the method according to any one of the above [1] to [7], and
   reacting a secondary amine compound and the carbonyl halide.
[12] A method for producing an amino acid N-carboxy anhydride,
   the method comprising the steps of:
   producing a carbonyl halide by the method according to any one of the above [1] to [7], and
   reacting an amino acid compound represented by the following formula (VII) and the carbonyl halide,
   wherein the amino acid N-carboxy anhydride is represented by the following formula (VIII): 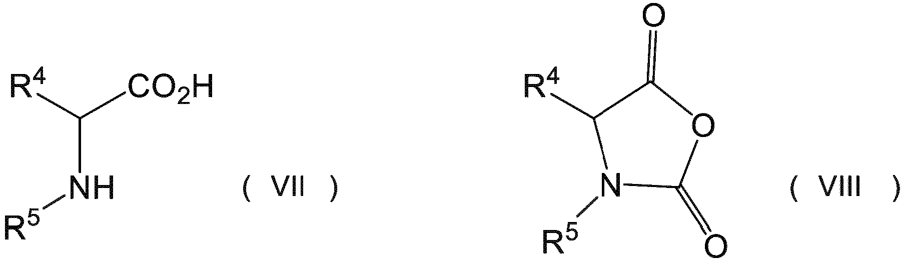 wherein
      R⁴ is an amino acid side chain group wherein a reactive group of the amino acid side chain group is protected,
      R⁵ is H or P¹-[-NH-CHR⁶-C(=O)-]ₗ- wherein R⁶ is an amino acid side chain group wherein a reactive group of the amino acid side chain group is protected, P¹ is a protective group of the amino group, l is an integer of 1 or more, a plurality of R⁶ is the same as or different from each other in the case where l is an integer of 2 or more.
[13] A method for producing a Vilsmeier reagent,
   wherein the Vilsmeier reagent is a salt represented by the following formula (X): 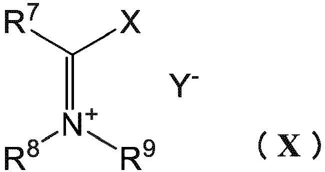 wherein
   R⁷ is a hydrogen atom, a C₁₋₆ alkyl group or a C₆₋₁₂ aromatic hydrocarbon group optionally having a substituent group,
   R⁸ and R⁹ are independently a C₁₋₆ alkyl group or a C₆₋₁₂ aromatic hydrocarbon group optionally having a substituent group, or R⁸ and R⁹ may form a 4 or more-membered and 7 or less-membered ring structure together with each other,
   X is a halogeno group selected from the group consisting of chloro, bromo and iodo,
   Y⁻ is a counter anion,
   the method comprising the steps of:
      producing a carbonyl halide by the method according to any one of the above [1] to [7], and
      reacting the carbonyl halide and an amide compound represented by the following formula (IX): 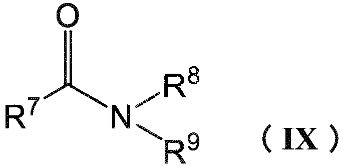
      wherein R⁷ to R⁹ have the same meanings as the above.

### EFFECT OF THE INVENTION

A useful carbonyl halide can be produced by the present invention method safely and efficiently from a halomethane as a raw material compound, while a halomethane applies a high load on the environment and thus the use and disposal thereof are restricted. In addition, though a halomethane product may contain a stabilizing agent and such a stabilizing agent may inhibit an oxidative photolytic degradation reaction, ozone used in the present invention method may decompose the stabilizing agent and promote the oxidative photolytic degradation reaction of the halomethane. Furthermore, a highly corrosive and toxic halogen gas such as chlorine gas is not needed to be used in the present invention method. The present invention is therefore industrially very useful as the technology to enable the effective utilization of a halomethane and the efficient production of a carbonyl halide such as carbonyl chloride.

### BRIEF DESCRIPTION OF THE DRAWINGS

[Figure 1] Figure 1 is a schematic diagram to demonstrate one example of the constitution of a reaction system usable in the present invention.
[Figure 2] Figure 2 is a schematic diagram to demonstrate one example of the constitution of a reaction system usable in the present invention.
[Figure 3] Figure 3 is a schematic diagram to demonstrate one example of the constitution of a reaction system usable in the present invention.
[Figure 4] Figure 4 is a schematic diagram to demonstrate one example of the constitution of a reaction system usable in the present invention.

### MODE FOR CARRYING OUT THE INVENTION

The present invention method is hereinafter described step by step, and the present invention is not restricted to the following specific examples.

### 1. Oxidative photolytic degradation step

A halomethane is oxidatively photolyzed to produce a carbonyl halide in this step by irradiating a light to the halomethane having 1 or more halogeno groups selected from the group consisting of chloro, bromo and iodo in the presence of oxygen and ozone.

The halomethane used in the present invention means methane having 1 or more halogeno groups selected from the group consisting of chloro, bromo and iodo. The halomethane may be transformed into a carbonyl halide by decomposing the halomethane with oxygen, ozone and light energy.

A halomethane is oxidatively photolyzed and may play a similar role to a carbonyl halide in the present invention as described above. A halomethane is preferably a poly halomethane having 2 or more halogeno groups, and is also preferably a perhalomethane, of which all of the hydrogen atoms are substituted with halogeno groups.

An example of the halomethane specifically includes a chloromethane such as dichloromethane, chloroform and carbon tetrachloride; a bromomethane such as dibromomethane and bromoform; an iodomethane such as iodomethane and diiodomethane; a halomethane having 2 or more kinds of a halogeno group, such as bromochloromethane, chloroiodomethane, bromoiodomethane and bromochloroiodomethane.

The halomethane may be appropriately selected depending on the target chemical reaction and the desired product. Only one kind of the halomethane may be used, or 2 or more kinds of the halomethanes may be used in combination. Only one kind of the halomethane is preferably used depending on the target product compound. The halomethane having chloro is preferred and chloroform is more preferred in terms of vaporization and a cost.

A general halomethane product contains a stabilizing agent such as amylene and ethanol, which inhibits the decomposition of a halomethane. Since a halomethane is oxidatively photolyzed in the present invention, a halomethane from which a stabilizing agent is removed may be used. When a halomethane from which a stabilizing agent is removed is used, a halomethane may be decomposed more efficiently; for example, a visible light having a relatively low energy may be used, and a time for irradiating a light may be reduced. A method for removing a stabilizing agent from a halomethane is particularly not restricted. For example, a halomethane may be washed with water to remove a hydrophilic stabilizing agent and then dried. Since ozone is used and thus a stabilizing agent may be decomposed by ozone, a halomethane containing a stabilizing agent may be directly used in the present invention method.

In particular, chloroform, which is an inexpensive general purpose solvent, can be used as the halomethane used in the present invention method. In addition, for example, the halomethane which has been used as a solvent can be recovered to be reused. Such a used halomethane is preferably purified to some extent for use, since if a large amount of an impurity and water are contained, the reaction may be possibly inhibited. For example, it is preferred that water and a water-soluble impurity are removed by washing a used halomethane with water and then the halomethane is dried over anhydrous sodium sulfate, anhydrous magnesium sulfate or the like. Excessive purification by which the productivity becomes less is not needed, since the reaction may proceed even in the case where about 1 mass% of water is contained. The water content is more preferably 0.5 mass% or less, even more preferably 0.2 mass% or less, and even more preferably 0.1 mass% or less. The water content is preferably 0 mass% or the detection limit or less. The above-described halomethane to be reused may contain a degradant of the halomethane.

In particular, when a halomethane is not a liquid under atmospheric temperature and atmospheric pressure or is hardly vaporized, a solvent may be used in addition to the halomethane. A solvent may accelerate the decomposition of a halomethane. Furthermore, a solvent may suppress the decomposition of a carbonyl halide produced by the oxidative photolytic decomposition of a halomethane. Such a solvent preferably appropriately dissolve a halomethane. An example of the solvent includes a ketone solvent such as acetone, methyl ethyl ketone, methyl isobutyl ketone and cyclohexanone; an aliphatic hydrocarbon solvent such as n-hexane; an aromatic hydrocarbon solvent such as benzene, toluene, xylene and benzonitrile; an ether solvent such as diethyl ether, tetrahydrofuran and dioxane; and a nitrile solvent such as acetonitrile.

A supply amount of oxygen may be appropriately adjusted in the range in which a halomethane can be oxidatively photolyzed efficiently. When oxygen is blown into a liquid halomethane, for example, oxygen may be supplied in an amount of 10 mL/min or more and 500 mL/min or less and 0.1 mmol/min or more and 50 mmol/min or less per 100 mL of a halomethane. The rate is preferably 20 mL/min or more, more preferably 50 mL/min or more, and preferably 300 mL/min or less or 200 mL/min or less, more preferably 100 mL/min or less, preferably 0.5 mmol/min or more, more preferably 1 mmol/min or more, preferably 30 mmol/min or less or 20 mmol/min or less, more preferably 10 mmol/min or less or 5 mmol/min or less.

When a gaseous halomethane and oxygen are mixed, for example, a volume ratio of oxygen to the halomethane may be adjusted to 0.1 or more and 5 or less, and a molar ratio of oxygen to the halomethane may be adjusted to 0.1 or more and 5 or less. The volume ratio and the molar ratio are preferably 0.2 or more, more preferably 0.5 or more, and preferably 3 or less or 2 or less, more preferably 1.5 or less.

The reaction for the production of a carbonyl halide by oxidative photolysis of a halomethane can be accelerated and a polymer having a higher molecular weight may be produced using the produced carbonyl halide by using ozone in the present invention in comparison with the case where only oxygen is used, though the reaction mechanism is unknown since ozone is not a radical substance. In addition, the stabilizing agent of a halomethane, such as amylene, may be decomposed by ozone. Furthermore, ozone may inhibit the coloration of the reaction mixture and the target compound by decomposing the colored component or preventing the production thereof. When oxygen is blown into a liquid halomethane, for example, the supply amount of ozone can be adjusted to 0.5 mL/min or more and 50 mL/min or less, 0.005 mmol/min or more and 5 mmol/min or less per 100 mL of the halomethane. When the ratio of ozone to a halomethane is higher, the oxidative photolytic reaction of a halomethane can proceed successfully. On the one hand, when the ratio of ozone to a halomethane is lower, the further photolysis of the produced carbonyl halide may be inhibited more surely. The above-described ratio is preferably 1 mL/min or more, more preferably 2 mL/min or more, and preferably 30 mL/min or less or 20 mL/min or less, more preferably 10 mL/min or less, and preferably 0.01 mmol/min or more, more preferably 0.05 mmol/min or more, and preferably 3 mmol/min or less or 2 mmol/min or less, more preferably 1 mmol/min or less or 0.5 mmol/min or less.

When a gaseous halomethane and ozone are mixed, for example, the volume ratio of ozone to the halomethane can be adjusted to 0.005 or more and 0.5 or less, and a molar ratio of ozone to the halomethane can be adjusted to 0.005 or more and 0.5 or less. The above-described volume ratio and molar ratio are preferably 0.01 or more, more preferably 0.05 or more, and preferably 0.3 or less or 0.2 or less, more preferably 0.15 or less.

When a gaseous halomethane and ozone are mixed, for example, a volume ratio of the halomethane to ozone can be adjusted to 0.01 times or more and 100 times or less. The ratio is preferably 0.05 or more, more preferably 0.1 or more or 1 or more, and preferably 50 times or less or 20 times or less.

Ratios of oxygen and ozone may be also appropriately adjusted. For example, a ratio of ozone to the total of oxygen and ozone can be adjusted to 1 vol% or more and 50 vol% or less. The ratio is preferably 30 vol% or less and more preferably 20 vol% or less.

An inert gas such as nitrogen and argon may be mixed in addition to oxygen and ozone. Air can be used as an oxygen source. Air is advantageous in terms of a cost. Chlorine is preferably not used in the present invention. Chlorine may accelerate the oxidative photolysis of a halomethane, but a specially equipped production facility is needed due to strong corrosive property and toxicity of chlorine. Ozone is used in the present invention to accelerate the oxidative photolytic reaction of a halomethane, since ozone is safer than chlorine.

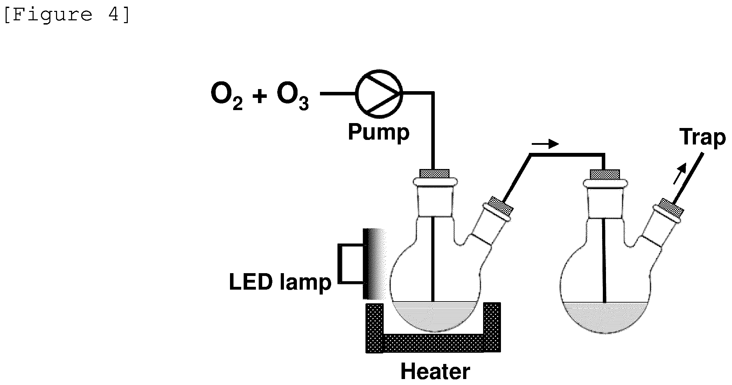

A light is irradiated to a halomethane in the presence of oxygen and ozone in this step. The phrase, "in the presence of oxygen and ozone", means any one of the condition that oxygen and ozone are dissolved in a liquid halomethane and the condition that a gaseous halomethane, oxygen and ozone are mixed to be contacted with each other. For example, a mixed gas containing oxygen and ozone may be supplied into a composition containing a halomethane by bubbling as schematically demonstrated in Figure 4. The composition containing a halomethane may be the halomethane only.

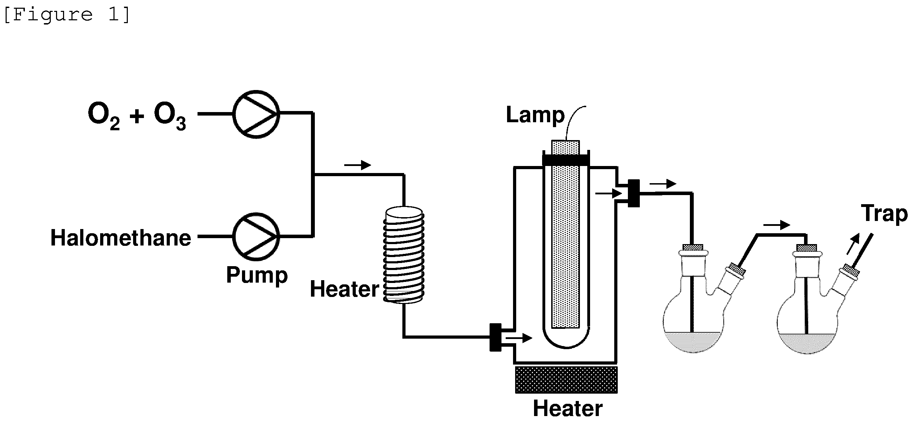

Alternatively, a mixed gas containing a gaseous halomethane, oxygen and ozone is prepared, and a light may be irradiated to the mixed gas. The oxidative photolysis of a halomethane may proceed more efficiently by irradiating a light to the mixed gas. The condition to prepare the mixed gas is particularly not restricted; for example, a halomethane, oxygen and ozone are mixed and supplied to a heater to be vaporized by heating at the boiling point of the halomethane or higher, and the gas may be supplied to a reactor as demonstrated in Figures 1 to 3. The upper limit of the temperature for heating a halomethane is particularly not restricted as long as the halomethane is successfully vaporized, and for example, the temperature can be adjusted to the boiling point + 50°C.

A halomethane may be oxidatively photolyzed in a two-phase system having a liquid phase and a gas phase. For example, a light may be irradiated to a liquid phase and a gas phase while a liquid halomethane is heated using a heater at the boiling point - 10°C or higher and lower than the boiling point in the reaction system demonstrated in Figure 4. The vaporization of a halomethane is accelerated at the time by blowing a mixed gas containing oxygen and ozone into a composition containing the halomethane to bubble the composition. A mixture containing a halomethane may be stirred to further accelerate the vaporization of the halomethane.

A light containing a high energy light such as a short-wavelength light can be used as a light to be irradiated to a halomethane. For example, a light containing ultraviolet light can be used. A halomethane may be oxidatively photolyzed with efficiency by using a high energy light containing a short-wavelength light. Specifically, a light containing a light having a wavelength of 180 nm or more and 500 nm or less, and a light having a peak wavelength included in a range of 180 nm or more and 500 nm or less can be used. The wavelength of a light to be irradiated may be appropriately determined, a light having a wavelength of 400 nm or less can be used, a light having a wavelength of 300 nm or less can be used, and a light having a peak wavelength included in the above ranges can be also used. For example, a light containing UV-B having a wavelength of 280 nm or more and 315 nm or less and/or UV-C having a wavelength of 180 nm or more and 280 nm or less can be used, a light containing UV-C having a wavelength of 180 nm or more and 280 nm or less can be used, and a light having a peak wavelength included in the above ranges can be also used.

A halomethane may be oxidatively photolyzed even by using a light having relatively low energy by the present invention, since a halomethane is oxidatively photolyzed in the presence of ozone. An example of an irradiation light having relatively low energy includes a light having a peak wavelength included in a range of a visible wavelength light. An example of a wavelength range of a light having relatively low energy includes 250 nm or more and 830 nm or less. The wavelength is preferably 280 nm or more, more preferably 300 nm or more, and preferably 800 nm or less or 700 nm or less, more preferably 600 nm or less or 500 nm or less, even more preferably 400 nm or less. A light having a peak wavelength included in the above ranges is also preferred.

A means for irradiating a light is particularly not restricted as long as a light having the above-described wavelength can be irradiated by the means. An example of a light source containing a light having a wavelength included in the ranges includes sunlight, low pressure mercury lamp, medium pressure mercury lamp, high pressure mercury lamp, ultrahigh pressure mercury lamp, chemical lamp, black light lamp, metal halide lamp and LED lamp. A low pressure mercury lamp and an LED lamp are preferred, and an LED lamp is more preferred in terms of a reaction efficiency and a cost.

The condition such as a strength of the irradiation light may be appropriately determined depending on the kind of a halomethane. For example, a light strength at a shortest distance position of a liquid or gaseous halomethane from the light source is preferably 1 mW/cm² or more and 500 mW/cm² or less depending on an implementation scale and a wavelength of an irradiation light. When a wavelength of an irradiation light is relatively short, for example, the light strength is more preferably 100 mW/cm² or less or 50 mW/cm² or less and even more preferably 20 mW/cm² or less or 10 mW/cm² or less. When a wavelength of an irradiation light is relatively long, the light strength is more preferably 10 mW/cm² or more or 20 mW/cm² or more, may be 50 mW/cm² or more or 100 mW/cm² or more, and more preferably 400 mW/cm² or less or 300 mW/cm² or less, even more preferably 200 mW/cm² or less. The shortest distance between a light source and a halomethane is preferably 1 m or less, more preferably 50 cm or less, and even more preferably 10 cm or less or 5 cm or less. The lower limit of the shortest distance is particularly not restricted and may be 0 cm, in other words, the light source may be immersed in a liquid or gaseous halomethane.

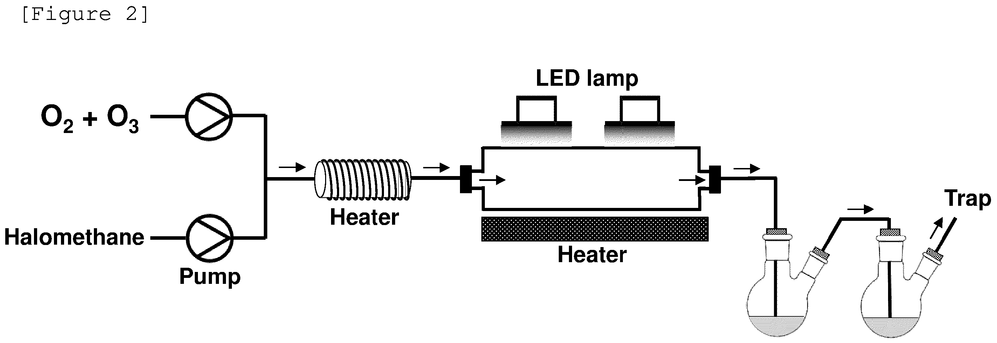

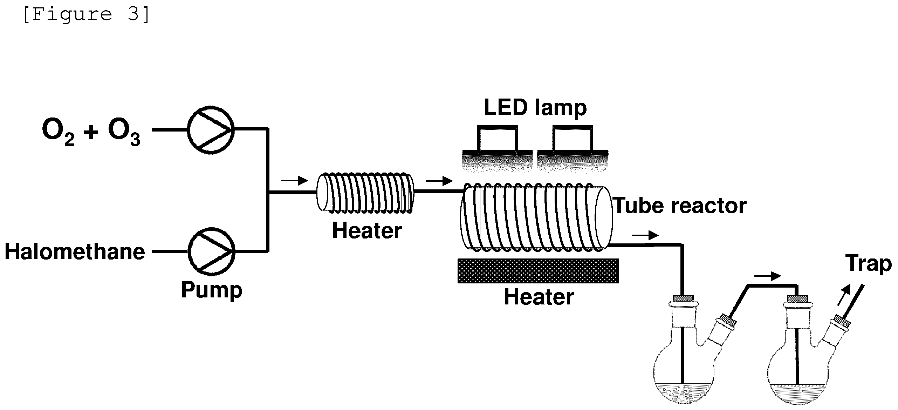

An embodiment to irradiate a light to a halomethane is particularly not restricted. For example, a light source may be placed in a reactor in which there is a liquid or gaseous halomethane as demonstrated in Figure 1 and Figure 4. In addition, 1 or more light sources may be placed around a transparent reactor in which there is a liquid or gaseous halomethane as demonstrated in Figure 2 and Figure 3.

A halomethane may be oxidatively photolyzed into a carbonyl halide by oxygen, ozone and an irradiation light. It has been also known that a carbonyl halide is decomposed by a high energy light particularly. Thus, it is important to adjust the condition to irradiate a light in order not to excessively decompose the produced carbonyl halide.

For example, the time to irradiate a light to a halomethane is preferably 1 second or more and 5000 seconds or less, though the time should be adjusted depending on the wavelength of an irradiation light and the reaction temperature. The time to irradiate a light to a flowing mixed gas containing a halomethane, oxygen and ozone means a retention time of the flowing mixed gas in a photoreaction vessel for continuously irradiating a light to the mixed gas. When the time is 1 second or more, a halomethane can be oxidatively photolyzed more surely. When the time is 5000 seconds or less, the excessive decomposition of the produced carbonyl halide can be inhibited more surely. The time is preferably 5 seconds or more or 10 seconds or more, more preferably 30 seconds or more or 60 seconds or more, even more preferably 120 seconds or more or 600 seconds or more, and preferably 3000 seconds or less, more preferably 2000 seconds or less, even more preferably 1000 seconds or less. A relatively low energy light having a peak wavelength of 300 nm or more can be also used to inhibit the decomposition of a carbonyl halide.

A flow rate of a flowing mixed gas in a photoreaction vessel to irradiate a high energy light to the flowing mixed gas is preferably determined in consideration of the inside volume of the photoreaction vessel. When the inside volume of a photoreaction vessel is large, for example, the flow rate is adjusted to be fast, since the retention time of the mixed gas tends to be longer. When the inside volume of a photoreaction vessel is small, the flow rate of the mixed gas is preferably adjusted to be slow. Specifically, the flow rate of the flowing mixed gas can be determined in consideration of the desired retention time and the inside volume of a photoreaction vessel, since a ratio of [inside volume of photoreaction vessel (L)]/[flow rate of flowing mixed gas (L/sec)] corresponds to a retention time (sec) of the flowing mixed gas in a photoreaction vessel. A linear velocity of the flowing mixed gas in a photoreaction vessel can be adjusted to about 0.001 m/min or more and 100 m/min or less. When the linear velocity is 0.001 m/min or more, the photolytic decomposition of a carbonyl halide produced from a halomethane by a gas phase reaction can be inhibited more surely. When the linear velocity is 100 m/min or less, a sufficient time to produce a carbonyl halide from a halomethane can be ensured more surely. The linear velocity can be calculated by dividing the velocity of the flowing mixed gas through a photoreaction vessel by the cross-sectional area of the photoreaction vessel. When the cross-sectional area of a photoreaction vessel is not constant, the cross-sectional area may be regarded as the average value of the cross-sectional areas of the photoreaction vessel in the moving direction of the flowing mixed gas. The average value can be calculated by dividing the volume of a photoreaction vessel by the length of the photoreaction vessel in the moving direction of the flowing mixed gas. The linear velocity is preferably 0.01 m/min or more, and preferably 50 m/min or less or 20 m/min or less, more preferably 10 m/min or less or 5 m/min or less, and even more preferably 1 m/min or less or 0.5 m/min or less.

The temperature during the irradiation of a light to a halomethane may be appropriately adjusted in the range in which the halomethane can be oxidatively photolyzed and the excessive decomposition of the produced carbonyl halide can be inhibited. For example, the temperature can be adjusted to 35°C or higher and 250°C or lower. The temperature is preferably 40°C or higher or 50°C or higher, more preferably 60°C or higher or 70°C or higher, even more preferably 75°C or higher, and preferably 200°C or lower, more preferably 150°C or lower, even more preferably 120°C or lower. The temperature can be adjusted by a heating medium, or a heater installed on a reactor.

When a light is irradiated to a mixed gas containing a halomethane, oxygen and ozone, the mixed gas may not be pressured or may be pressured at least to such an extent that the mixed gas can be passed through a reaction vessel. In addition, the productivity may be improved by pressuring the mixed gas. The gauge pressure of the mixed gas in a reaction vessel may be adjusted to 0 MPaG or more and 2 MPaG or less, and is preferably 1 MPaG or less and more preferably 0.5 MPaG or less.

A halomethane is oxidatively photolyzed by this step to produce a carbonyl halide [X-C(=O)-X wherein X is 1 or more halogeno groups selected from the group consisting of chloro, bromo and iodo]. In addition, not only a carbonyl halide but also a carbonyl halide-like compound which plays a similar role to a carbonyl halide may be produced. The carbonyl halide produced by the present invention contains such a carbonyl halide-like compound. Typical examples of reactions using the carbonyl halide is hereinafter described.

### 3. Downstream reaction step - Production of carbonate compound

A carbonate compound can be produced by reacting the carbonyl halide with an alcohol compound.

The embodiment of the reaction is particularly not restricted. For example, the gas containing the produced carbonyl halide may be blown into a composition containing an alcohol compound as demonstrated in Figures 1 to 4. In addition, for example, a light is irradiated to a composition containing a halomethane and an alcohol compound while oxygen and ozone are blown thereinto in the reaction system demonstrated in Figure 4; as a result, the carbonyl halide produced in the composition can be immediately reacted with the alcohol compound. Alternatively, the carbonyl halide produced in a gas phase can be immediately reacted with an alcohol compound by continuously supplying the alcohol compound with a halomethane to the gas phase.

A cooler may be installed between a photoreactor and a reactor for the reaction with a reactant such as an alcohol compound. The temperature of the cooler is preferably adjusted in the range that the produced carbonyl halide can pass therethrough. For example, the temperature of a cooler is preferably adjusted to 10°C or higher and lower than an atmospheric temperature in the case where carbonyl chloride is produced, since the boiling point of carbonyl chloride [ClC(=O)Cl] among a carbonyl halide is 8.2°C. A carbonyl halide is also referred to as a carbonyl dihalide, and carbonyl chloride is also referred to as carbonyl dichloride.

An alcohol compound is an organic compound having a hydroxy group. For example, an example of an alcohol compound includes a monovalent alcohol compound represented by the following formula (I) and a divalent alcohol compound represented by the following formula (II). Hereinafter, a compound represented by the formula x is abbreviated as "compound x" in some cases. For example, a "monovalent alcohol compound represented by the formula (I)" is abbreviated as the "monovalent alcohol compound (I)" in some cases.

R¹-OH ··· (I)

HO-R²-OH ··· (II)

wherein R¹ is a monovalent organic group and R² is a divalent organic group.

The organic group is particularly not restricted as long as the organic group is inert toward the reaction of this step. For example, an example of the organic group includes a C₁₋₁₀ aliphatic hydrocarbon group optionally having a substituent group, a C₆₋₃₀ aromatic hydrocarbon group optionally having a substituent group, a heteroaryl optionally having a substituent group, an organic group formed by binding 2 or more and 5 or less of a C₁₋₁₀ aliphatic hydrocarbon group optionally having substituent group and a C₆₋₃₀ aromatic hydrocarbon group optionally having a substituent group, and an organic group formed by binding 2 or more and 5 or less of a C₁₋₁₀ aliphatic hydrocarbon group optionally having a substituent group and a heteroaryl group optionally having a substituent group.

For example, an example of the C₁₋₁₀ aliphatic hydrocarbon group includes a C₁₋₁₀ linear aliphatic hydrocarbon group, a C₃₋₁₀ cyclic aliphatic hydrocarbon group, and an organic group formed by binding 2 or more and 5 or less of a C₁₋₁₀ linear aliphatic hydrocarbon group and a C₃₋₁₀ cyclic aliphatic hydrocarbon group.

The "C₁₋₁₀ linear aliphatic hydrocarbon group" means a linear or branched saturated or unsaturated aliphatic hydrocarbon group having a carbon number of 1 or more and 10 or less. For example, an example of the monovalent C₁₋₁₀ linear aliphatic hydrocarbon group includes a C₁₋₁₀ alkyl group, a C₂₋₁₀ alkenyl group and a C₂₋₁₀ alkynyl group.

An example of a C₁₋₁₀ alkyl group includes methyl, ethyl, n-propyl, isopropyl, n-butyl, 1-methylpropyl, 2-methylpropyl, 1,1-dimethylethyl, 2,2-dimethylethyl, n-pentyl, n-hexyl, 2-hexyl, 3-hexyl, 4-methyl-2-pentyl, n-heptyl, n-octyl and n-decyl. The C₁₋₁₀ alkyl group is preferably a C₂₋₈ alkyl group and more preferably a C₄₋₆ alkyl group.

An example of a C₂₋₁₀ alkenyl group includes ethenyl (vinyl), 1-propenyl, 2-propenyl (allyl), butenyl, hexenyl, octenyl and decenyl. The C₂₋₁₀ alkenyl group is preferably a C₂₋₈ alkenyl group and more preferably a C₄₋₆ alkenyl group.

An example of a C₂₋₁₀ alkynyl group includes ethyny, propynyl, butynyl, hexynyl, octynyl and pentadecynyl. The C₂₋₁₀ alkynyl group is preferably a C₂₋₈ alkynyl group and more preferably a C₂₋₆ alkynyl group.

The "C₃₋₁₀ cyclic aliphatic hydrocarbon group" means a cyclic saturated or unsaturated aliphatic hydrocarbon group having a carbon number of 3 or more and 10 or less. For example, an example of a monovalent C₃₋₁₀ cyclic aliphatic hydrocarbon group includes a C₃₋₁₀ cycloalkyl group, a C₄₋₁₀ cycloalkenyl group and a C₄₋₁₀ cycloalkynyl group.

An example of the organic group formed by binding 2 or more and 5 or less of a C₁₋₁₀ linear aliphatic hydrocarbon group and a C₃₋₁₀ cyclic aliphatic hydrocarbon group includes a monovalent C₃₋₁₀ cyclic aliphatic hydrocarbon group-divalent C₁₋₁₀ linear aliphatic hydrocarbon group and a monovalent C₁₋₁₀ linear aliphatic hydrocarbon group-divalent C₃₋₁₀ cyclic aliphatic hydrocarbon group-divalent C₁₋₁₀ linear aliphatic hydrocarbon group.

The "C₆₋₁₂ aromatic hydrocarbon group" means an aromatic hydrocarbon group having a carbon number of 6 or more and 12 or less. For example, an example of a monovalent C₆₋₁₂ aromatic hydrocarbon group includes phenyl, indenyl, naphthyl and biphenyl, and a monovalent C₆₋₁₂ aromatic hydrocarbon group is preferably phenyl.

The "C₆₋₃₀ aromatic hydrocarbon group" means an aromatic hydrocarbon group having a carbon number of 6 or more and 30 or less. For example, an example of a divalent C₆₋₃₀ aromatic hydrocarbon group includes the alcohol compound (II-1) described later in addition to a divalent C₆₋₁₂ aromatic hydrocarbon group such as phenylene, indenylene, naphthylene and biphenylene.

The "heteroaryl group" means a 5-membered aromatic heterocyclic group, a 6-membered aromatic heterocyclic group and a condensed aromatic heterocyclic group having at least 1 hetero atom such as a nitrogen atom, an oxygen atom and a sulfur atom. An example of a heteroaryl group includes a monovalent 5-membered heteroaryl group such as pyrrolyl, imidazolyl, pyrazolyl, thienyl, furyl, oxazolyl, isoxazolyl, thiazolyl, isothiazolyl and thiadiazole; a monovalent 6-membered heteroaryl group such as pyridinyl, pyrazinyl, pyrimidinyl and pyridazinyl; and a monovalent condensed aromatic heterocyclic group such as indolyl, isoindolyl, quinolinyl, isoquinolinyl, benzofuranyl, isobenzofuranyl and chromenyl.

An example of the "organic group formed by binding 2 or more and 5 or less of a C₁₋₁₀ aliphatic hydrocarbon group and a C₆₋₁₂ aromatic hydrocarbon group" includes a C₆₋₁₂ aromatic hydrocarbon group-C₁₋₁₀ linear aliphatic hydrocarbon group, a C₁₋₁₀ linear aliphatic hydrocarbon group-C₆-₁₂ aromatic hydrocarbon group, a C₁₋₁₀ linear aliphatic hydrocarbon group-C₆₋₁₂ aromatic hydrocarbon group-C₁₋₁₀ linear aliphatic hydrocarbon group and a C₆₋₁₂ aromatic hydrocarbon group-C₁₋₁₀ linear aliphatic hydrocarbon group-C₆-₁₂ aromatic hydrocarbon group. An example of the "organic group formed by 2 or more and 5 or less of a C₁₋₁₀ aliphatic hydrocarbon group and a heteroaryl group" includes a heteroaryl group-C₁₋₁₀ linear aliphatic hydrocarbon group, a C₁₋₁₀ linear aliphatic hydrocarbon group-heteroaryl group, a C₁₋₁₀ linear aliphatic hydrocarbon group-heteroaryl group-C₁₋₁₀ linear aliphatic hydrocarbon group and a heteroaryl group-C₁₋₁₀ linear aliphatic hydrocarbon group-heteroaryl group.

A C₁₋₁₀ aliphatic hydrocarbon group may optionally have a substituent group, and an example of the substituent group includes 1 or more substituent groups selected from the group consisting of a halogeno group, a nitro group and cyano group. The substituent group is preferably a halogeno group. A C₆₋₁₂ aromatic hydrocarbon group and a heteroaryl group may have a substituent group, and an example of the substituent group includes 1 or more substituent groups selected from the group consisting of a C₁-₆ alkyl group, a C₁₋₆ alkoxy group, a halogeno group, a nitro group and a cyano group. The substituent group is preferably a halogeno group. An example of the "halogeno group" includes fluoro, chloro, bromo and iodo, and the halogeno group is preferably fluoro.

The alcohol compound may be classified into a fluorinated alcohol compound essentially having a fluoro group as a substituent group and a non-fluorinated alcohol compound that is not substituted with a fluoro group. The non-fluorinated alcohol compound may have a halogeno group as a substituent group, and the halogeno group is 1 or more halogeno groups selected from chloro, bromo and iodo. The group "R^{x}" having a fluoro group as a substituent group may described as "R_{F}^{x}".

The "C₁₋₆ alkyl group" is a linear or branched monovalent saturated aliphatic hydrocarbon group having a carbon number of 1 or more and 6 or less. An example of the C₁₋₆ alkyl group includes methyl, ethyl, n-propyl, isopropyl, n-butyl, isobutyl, s-butyl, t-butyl, n-pentyl and n-hexyl. The C₁₋₆ alkyl group is preferably a C₁₋₄ alkyl group, more preferably a C₁₋₂ alkyl group, and even more preferably methyl.

The "C₁₋₆ alkoxy group" means a linear or branched saturated aliphatic hydrocarbon oxy group having a carbon number of 1 or more and 6 or less. An example of the C₁₋₆ alkoxy group includes methoxy, ethoxy, n-propoxy, isopropoxy, n-butoxy, isobutoxy, t-butoxy, n-pentoxy and n-hexoxy. The C₁₋₆ alkoxy group is preferably a C₁₋₄ alkoxy group, more preferably a C₁₋₂ alkoxy group and even more preferably methoxy.

The monovalent alcohol compound (I) may be a fluorinated alcohol compound. An example of the monovalent fluorinated alcohol compound (I) includes a fluorinated ethanol such as difluoroethanol and trifluoroethanol; and a fluorinated propanol such as monofluoropropanol, difluoropropanol, trifluoropropanol, tetrafluoropropanol, pentafluoropropanol and hexafluoropropanol.

An example of a divalent organic group includes a divalent organic group that is a counterpart to the examples of the monovalent organic group. For example, the divalent organic group that is a counterpart to a C₁₋₁₀ alkyl group, a C₂₋₁₀ alkenyl group and a C₂₋₁₀ alkynyl group as a monovalent organic group is a C₁₋₁₀ alkane-diyl group, a C₂₋₁₀ alkene-diyl group and a C₂₋₁₀ alkyne-diyl group.

The divalent organic group may be a divalent (poly)alkylene glycol group: -[-O-R²-]ₙ- wherein R² is a C₁₋₈ alkane-diyl group and n is an integer of 1 or more and 50 or less.

For example, an example of the divalent alcohol compound (II) includes the following divalent alcohol compound (II-1): 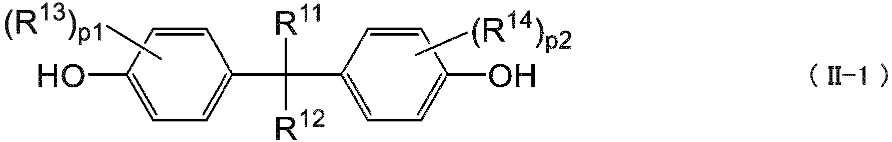 wherein
R¹¹ and R¹² are independently H, a C₁₋₆ alkyl group, a C₁₋₆ fluoroalkyl group or a C₆₋₁₂ aromatic hydrocarbon group, or R¹¹ and R¹² form a C₃₋₆ cycloalkyl optionally substituted with a C₁₋₆ alkyl together with each other,
R¹³ and R¹⁴ are independently H, a C₁₋₆ alkyl group or a C₆₋₁₂ aromatic hydrocarbon group, and when P¹ or p² is an integer of 2 or more, a plurality of R¹³ or R¹⁴ may be the same as or different from each other,
P¹ and p² are independently an integer of 0 or more and 4 or less.

An example of the divalent alcohol compound (II-1) specifically includes 2,2-bis(4-hydroxyphenyl)propane, 1,1-bis(4-hydroxyphenyl)-1-phenylethane, 2,2-bis(4-hydroxyphenyl)hexafluoropropane, 2,2-bis(4-hydroxyphenyl)butane, bis(4-hydroxyphenyl)diphenylmethane, 2,2-bis(3-methyl-4-hydroxyphenyl)propane, 1,1-bis(4-hydroxyphenyl) ethane, bis(4-hydroxyphenyl)methane and 2,2-bis(4-hydroxy-3-isopropylphenyl)propane. The divalent alcohol compound (II-1) is preferably 2,2-bis(4-hydroxyphenyl)propane (Bisphenol A).

The divalent alcohol compound (II) may be a fluorinated alcohol compound. An example of the divalent fluorinated alcohol compound (II) includes a fluorinated ethylene glycol; a fluorinated propylene glycol such as monofluoropropylene glycol and difluoropropylene glycol; a fluorinated butanediol such as monofluorobutanediol, difluorobutanediol, trifluorobutanediol and tetrafluorobutanediol; a fluorinated pentanediol such as monofluoropentanediol, difluoropentanediol, trifluoropentanediol, tetrafluoropentanediol, pentafluoropentanediol and hexafluoropentanediol; a fluorinated hexanediol such as monofluorohexanediol, difluorohexanediol, trifluorohexanediol, tetrafluorohexanediol, pentafluorohexanediol, hexafluorohexanediol, heptafluorohexanediol and octafluorohexanediol; a fluorinated heptanediol such as monofluoroheptanediol, difluoroheptanediol, trifluoroheptanediol, tetrafluoroheptanediol, pentafluoroheptanediol, hexafluoroheptanediol, heptafluoroheptanediol, octafluoroheptanediol, nonafluoroheptanediol and decafluoroheptanediol; a fluorinated octanediol such as monofluorooctanediol, difluorooctanediol, trifluorooctanediol, tetrafluorooctanediol, pentafluorooctanediol, hexafluorooctanediol, heptafluorooctanediol, octafluorooctanediol, nonafluorooctanediol, decafluorooctanediol, undecafluorooctanediol and dodecafluorooctanediol; a fluorinated nonanediol such as monofluorononanediol, difluorononanediol, trifluorononanediol, tetrafluorononanediol, pentafluorononanediol, hexafluorononanediol, heptafluorononanediol, octafluorononanediol, nonafluorononanediol, decafluorononanediol, undecafluorononanediol, dodecafluorononanediol, tridecafluorononanediol and tetradecafluorononanediol; a fluorinated decanediol such as monofluorodecanediol, difluorodecanediol, trifluorodecanediol, tetrafluorodecanediol, pentafluorodecanediol, hexafluorodecanediol, heptafluorodecanediol, octafluorodecanediol, nonafluorodecanediol, decafluorodecanediol, undecafluorodecanediol, dodecafluorodecanediol, tridecafluorodecanediol, tetradecafluorodecanediol, pentadecafluorodecanediol and hexadecafluorodecanediol; and a fluorinated polyethylene glycol such as fluorinated diethylene glycol, fluorinated triethylene glycol, fluorinated tetraethylene glycol, fluorinated pentaethylene glycol and fluorinated hexaethylene glycol.

The usage amount of the alcohol compound may be appropriately adjusted as long as the reaction successfully proceeds, and the molar ratio of the alcohol compound to the produced carbonyl halide is preferably adjusted to (2/valence of alcohol compound) or more and (20/valence of alcohol compound) or less. For example, the divalent alcohol compound with a molar ratio of 1 or more to the produced carbonyl halide may be used, and the monovalent alcohol compound with the molar ratio of 2 or more may be used. The carbonate compound can be produced more efficiently by using an excessive amount of the alcohol compound. Since the yield of a carbonyl halide to the used halomethane is not constant, the molar ratio of the alcohol compound to the halomethane is preferably adjusted to (2/valence of alcohol compound) or more and (20/valence of alcohol compound) or less. For example, the molar ratio of the divalent alcohol compound to the halomethane is preferably adjusted to 1 or more, and the molar ratio of the monovalent alcohol compound to the halomethane is preferably adjusted to 2 or more. The molar ratio of the divalent alcohol compound is preferably 1.5 or more, more preferably 2 or more, and preferably 10 or less, preferably 5 or less. The molar ratio of the monovalent alcohol compound is preferably 2 or more, more preferably 4 or more, and preferably 20 or less, preferably 10 or less.

A base may be used for accelerating the reaction between the carbonyl halide and the alcohol compound. A base is classified into an inorganic base and an organic base. An example of an inorganic base includes a carbonate salt of an alkali metal, such as lithium carbonate, sodium carbonate, potassium carbonate and cesium carbonate; a carbonate salt of a group 2 metal, such as magnesium carbonate, calcium carbonate and barium carbonate; a hydrogencarbonate salt of an alkali metal, such as lithium hydrogencarbonate, sodium hydrogencarbonate, potassium hydrogencarbonate and cesium hydrogencarbonate; a hydroxide of an alkali metal, such as lithium hydroxide, sodium hydroxide and potassium hydroxide; a hydroxide of a group 2 metal, such as magnesium hydroxide and calcium hydroxide; and a fluoride salt of an alkali metal, such as lithium fluoride, sodium fluoride, potassium fluoride and cesium fluoride. A carbonate salt or a hydrogencarbonate salt of an alkali metal or a group 2 metal is preferred, and a carbonate salt of an alkali metal is more preferred due to relatively low hygroscopicity and deliquescence. When an inorganic base is used, fine inorganic base such as powder may be used, or an aqueous solution thereof may be used. An example of an organic base includes tri(C₁₋₄ alkyl)amine such as trimethylamine, triethylamine and diisopropylethylamine; a tert-butoxide of an alkali metal, such as sodium tert-butoxide and potassium tert-butoxide; and a non-nucleophilic organic base such as diazabicycloundecene, lithium diisopropylamide, lithium tetramethylpiperidine, 1,4-diazabicyclo[2.2.2]octane (DABCO), 1,5,7-triazabicyclo[4.4.0]deca-5-ene (TBD), 7-methyl-1,5,7-triazabicyclo[4.4.0]deca-5-ene (MTBD), 1,8-diazabicyclo[5.4.0]undeca-7-ene (DBU), 1,5-diazabicyclo[4.3.0]nona-5-ene (DBN), 1,1,3,3-tetramethylguanidine (TMG) and N-methylmorpholine in terms of the low reactivity with the product of a tetrahaloethylene by photoreaction. A low-nucleophilic organic base such as pyridine and lutidine may be also used.

A hydrogen halide such as hydrogen chloride is produced as a side product during an oxidative photolytic decomposition reaction of a halomethane and the reaction between a carbonyl halide and an alcohol compound. A base is useful for capturing such a hydrogen halide. But when a reaction tube having a small diameter, such as a coil reaction device, is used, a salt of a hydrogen halide and a base is produced and causes clogging. A base of which salt with a hydrogen halide is an ionic liquid is preferably used in such a case. An example of such a base includes an organic base such as an imidazole derivative, and an example of an imidazole derivative includes 1-methylimidazole. In addition, a base of which hydrochloride salt has a relatively low melting point, such as pyridine, may be also used.

The usage amount of a base may be appropriately adjusted as long as the reaction successfully proceeds and may be adjusted to, for example, 1 mol or more and 10 mol or less to 1 mol of the halomethane.

For example, a base may be preliminarily added into an alcohol compound or may be continuously injected with an alcohol compound.

When a carbonyl halide and an alcohol compound are reacted, a solvent may be used. For example, such a solvent may be added to the composition containing an alcohol compound. An example of the solvent includes a ketone solvent such as acetone, methyl ethyl ketone, methyl isobutyl ketone and cyclohexanone; an aliphatic hydrocarbon solvent such as n-hexane; an aromatic hydrocarbon solvent such as benzene, toluene, xylene and benzonitrile; an ether solvent such as diethyl ether, tetrahydrofuran and dioxane; a nitrile solvent such as acetonitrile; and a halogenated hydrocarbon solvent such as dichloromethane and chloroform.

The temperature for reacting a carbonyl halide and an alcohol compound is particularly not restricted and may be appropriately adjusted and may be adjusted to, for example, 0°C or higher and 250°C or lower. The temperature is more preferably 10°C or higher, even more preferably 20°C or higher, and more preferably 200°C or lower or 150°C or lower, even more preferably 100°C or lower or 80°C or lower. When a base is not used or a base is used for accelerating the reaction, the temperature may be adjusted to relatively high, for example, 50°C or higher or 100°C or higher.

The time to react the carbonyl halide with an alcohol compound is particularly not restricted and may be appropriately adjusted. For example, the time is preferably 0.5 hours or more and 50 hours or less. The reaction time is more preferably 1 hour or more, even more preferably 5 hours or more, and more preferably 30 hours or less, even more preferably 20 hours or less. For example, even after the production of a carbonyl halide is completed, the reaction mixture may be continuously stirred until the consumption of an alcohol compound is confirmed.

When the monovalent alcohol compound (I) is used for the reaction of the carbonyl halide and an alcohol compound, a linear carbonate compound represented by the following formula (III) is produced, and when a divalent alcohol compound (II) is used, the polycarbonate compound comprising the unit represented by the following formula (IV-1) or the cyclic carbonate compound represented by the following formula (IV-2) is produced. When the divalent alcohol compound (II) is used, whether the polycarbonate compound (IV-1) or the cyclic carbonate compound (IV-2) is produced, and the production ratio thereof depends on the distance between 2 hydroxy groups and the flexibility of the chemical structure of the divalent alcohol compound (II). Specifically, the matters may be predicted by a preliminary experiment.

R¹-O-C(=O)-O-R¹ (III)

[-A-R²-A-C(=O)-] (IV-1)

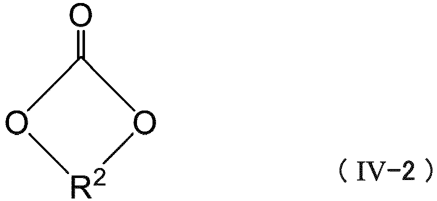

The polymerization reaction may proceed very efficiently and thus a polycarbonate compound having a large molecular weight may be produced by the present invention. For example, the weight-average molecular weight of the polycarbonate compound produced by the present invention method measured by gel permeation chromatography (GPC) in polystyrene equivalent is preferably 10,000 or more and 1,000,000 or less, and the number average molecular weight is 5,000 or more and 500,000 or less.

### 4. Downstream reaction step - Production of halogenated formate ester

A halogenated formate ester can be mainly produced without using a base and by adjusting a molar ratio of an alcohol compound to a halomethane to less than 1 in the above-described method for producing a carbonate compound. The molar ratio is preferably 0.9 or less and more preferably 0.8 or less. Both of a carbonate compound and a halogenated formate ester are produced depending on the conditions. The above-described monovalent alcohol compound (I) may be used as an alcohol compound. In addition, a halogenated formate fluorinated ester is produced from a fluorinated monovalent alcohol compound (I), and a halogenated formate non-fluorinated ester is produced from a non-fluorinated monovalent alcohol compound (I).

### 5. Downstream reaction step - Production of isocyanate compound

An isocyanate compound can be produced by reacting the carbonyl halide with a primary amine compound. An isocyanate compound is useful as a raw material of a carbamate compound, a urethane compound or the like. The conditions of the reaction may be similar to the conditions for the above-described method for producing a carbonate compound except that a primary amine compound is used in place of an alcohol compound except for the following point.

The primary amine compound is particularly not restricted as long as the compound has 1 or more amino groups (-NH₂ groups). For example, the primary amine compound (V): R³-(NH₂)ₘ can be used. In the formula, R³ is a m-valent organic group, and m is an integer of 1 or more and 6 or less, preferably 5 or less, 4 or less or 3 or less, more preferably 1 or 2, and even more preferably 2.

An example of the monovalent organic group among the organic group R³ includes the same group as the monovalent organic group R¹ in the above-described method for producing a carbonate compound, and an example of the divalent group includes the same group as the divalent organic group R². An example of the tri or more-valent organic group includes a tri or more-valent organic group counterpart to the examples of the monovalent organic group R¹. For example, the trivalent organic groups counterpart to a C₁₋₁₀ alkyl group, a C₂₋₁₀ alkenyl group and a C₂₋₁₀ alkynyl group are a C₁₋₁₀ alkane-triyl group, a C₂₋₁₀ alkene-triyl group and a C₂₋₁₀ alkyne-triyl group.

The isocyanate compound (VI): R³-(N=C=O)ₘ can be produced by the reaction of the carbonyl halide and the primary amine compound (V). But the urea compound: R³-[NH-C(=O)-NH-R³]ₘ may be possibly produced by reacting the produced R³-(N=C=O)ₘ with the primary amine compound (V). It is preferred in order to suppress such a reaction that the molar ratio of the primary amine compound (V) to a halomethane is adjusted to 1 or less, a salt is used as the primary amine compound (V) and a base is not used. In addition, an isocyanate compound can be efficiently produced by dissolving the produced carbonyl halide in a solvent to produce a carbonyl halide solution and adding the primary amine compound (V) or a solution thereof to the carbonyl halide solution with maintaining the molar ratio of the carbonyl halide to the primary amine compound (V) to more than 1.

When the target compound is an isocyanate compound, the molar ratio of the primary amine compound (V) to the produced carbonyl halide is preferably adjusted to 1 or less. But since it may be difficult in some cases that the amount of the produced carbonyl halide is accurately predicted, the molar ratio of the primary amine compound (V) to the used halomethane is preferably adjusted to less than 1. The molar ratio is preferably 0.5 or less, more preferably 0.2 or less, and preferably 0.001 or more, more preferably 0.05 or more. When the target compound is a urea compound, the ratio is preferably 2 or more, more preferably 4 or more, and preferably 20 or less, more preferably 15 or less.

A salt of the primary amine compound (V) is preferably used in the case where the target compound is an isocyanate compound, since an isocyanate compound is hardly reacted with an amine salt. An example of such a salt includes an inorganic acid salt such as hydrochloride salt, hydrobromide salt, hydroiodide salt, sulfate salt, nitrate salt, perchlorate salt and phosphate salt; and an organic acid salt such as oxalate salt, malonate salt, maleate salt, fumarate salt, lactate salt, malate salt, citrate salt, tartrate salt, benzoate salt, trifluoroacetate salt, acetate salt, methanesulfonate salt, p-toluenesulfonate salt and trifluoromethanesulfonate salt.

For example, the temperature for the reaction between the carbonyl halide and a primary amine compound is preferably adjusted to be lower than the temperature for the reaction with an alcohol compound in order to maintain the liquid condition of the carbonyl halide. For example, the reaction temperature may be adjusted to 15°C or lower, preferably 10°C or lower, more preferably 5°C or lower and even more preferably 2°C or lower. The lower limit of the temperature is particularly not restricted and is preferably adjusted to, for example, -80°C or higher and more preferably -20°C or higher or -15°C or higher.

When the target compound is an isocyanate compound with using a base, the base is preferably 1 or more bases selected from a heteroaryl amine and a non-nucleophilic strong base. A heteroaryl amine means a compound that has at least one heterocyclic ring and at least one amine functional group other than -NH₂. An example of the heteroaryl amine includes pyridine and a derivative thereof, such as pyridine, α-picoline, β-picoline, γ-picoline, 2,3-lutidine, 2,4-lutidine, 2,6-lutidine, 3,5-lutidine, 2-chloropyridine, 3-chloropyridine, 4-chloropyridine, 2,4,6-trimethylpyridine and 4-dimethylaminopyridine.

The "non-nucleophilic strong base" means a base of which nucleophilicity is weak but of which basicity is strong, since the basicity of the lone electron pair on the nitrogen atom is weak due to steric hindrance. An example of the non-nucleophilic strong base includes triethylamine, N,N-diisopropylethylamine, tripropylamine, triisopropylamine, tributylamine, tripentylamine, trihexylamine, triheptylamine, trioctylamine, tridecylamine, tridodecylamine, triphenylamine, tribenzylamine, N,N-diisopropylethylamine, 1,5,7-triazabicyclo[4.4.0]deca-5-ene (TBD), 7-methyl-1,5,7-triazabicyclo[4.4.0]deca-5-ene (MTBD), 1,8-diazabicyclo[5.4.0]undeca-7-ene (DBU), 1,5-diazabicyclo[4.3.0]nona-5-ene (DBN) and 1,1,3,3-tetramethylguanidine (TMG). In addition, a base of which basicity is relatively high may be used. For example, TBD (pK_{BH+}: 25.98), MTBD (pK_{BH+}: 25.44), DBU (pK_{BH+}: 24.33), DBN (pK_{BH+}: 23.89) and TMG (pK_{BH+}: 23.30) can be used as a base of which pK_{BH+}: basicity in acetonitrile is 20 or more.

In addition, a general organic amine such as trimethylamine, dimethylethylamine, diethylmethylamine, N-ethyl-N-methylbutylamine and 1-methylpyrrolidine may be also used as the base.

When the target compound is a urea compound, the molar ratio of the primary amine compound to the halomethane or the produced carbonyl halide is preferably adjusted to more than 1. The molar ratio is preferably 1.5 or more and more preferably 2 or more.

### 6. Downstream reaction step - Production of carbamoyl halide compound

A carbamoyl halide compound can be produced by reacting the carbonyl halide and a secondary amine compound. A carbamoyl halide compound is useful as a synthetic intermediate of a physiologically active substance such as a medicine and an agricultural chemical. An example of a physiologically active substance includes a carbamate ester such as a carbamate compound having a pesticidal activity. A secondary amine compound may be used in place of a primary amine compound in the above-described method for producing an isocyanate compound as a reaction embodiment.

The secondary amine compound is particularly not restricted as long as the compound has 1 or more secondary amino groups (-NHR groups). For example, the secondary amine compound (XI): R²¹-NH-R²² can be used. In the formula, R²¹ and R²² are independently monovalent organic groups, are exemplified by the same group as the monovalent organic R¹ in the above-described method for producing a carbonate compound, and are preferably a C₁₋₁₀ alkyl group.

The carbamoyl halide compound (XII): R²¹R²²N-C(=O)-X wherein X is 1 or more halogeno groups selected from the group consisting of chloro, bromo and iodo can be produced by reacting the carbonyl halide and the secondary amine compound (XI). But a urea compound: R²¹R²²N-C(=O)-NR²¹R²² may be possibly produced by reacting the produced R²¹R²²N-C(=O)-X and the secondary amine compound (XI). The usage amount of the secondary amine compound (XI) may be adjusted to relatively low in order to suppress such a reaction. For example, the molar ratio of the secondary amine compound (XI) to the methane is preferably adjusted to 1 or less. Alternatively, it is preferred not to use a base. In addition, a carbamoyl halide compound can be efficiently produced by dissolving the produced carbonyl halide in a solvent to prepare a carbonyl halide solution and adding the secondary amine compound (XI) or the solution thereof to the solution to adjust the molar ratio of the carbonyl halide to the secondary amine compound (XI) to more than 1.

When the target compound is a carbamoyl halide compound, the usage amount of the secondary amine compound (XI) is preferably adjusted to relatively low. For example, the molar ratio of the secondary amine compound (XI) to the produced carbonyl halide is preferably adjusted to 1 or less. But since it may be difficult in some cases to exactly predict the production amount of the carbonyl halide, the molar ratio of the secondary amine compound (XI) to the used methane is preferably adjusted to less than 1. The molar ratio is preferably 0.5 or less, more preferably 0.2 or less, and preferably 0.001 or more, more preferably 0.05 or more. When the target compound is a urea compound, the molar ratio is preferably 2 or more, more preferably 4 or more, and preferably 20 or less, more preferably 15 or less.

For example, the temperature for the reaction between the carbonyl halide and the secondary amine compound (XI) is preferably adjusted to lower than the reaction temperature with an alcohol compound in order to keep the liquid condition of the carbonyl halide. For example, the reaction temperature may be adjusted to 15°C or lower, preferably 10°C or lower, more preferably 5°C or lower, and even more preferably 2°C or lower. The lower limit of the temperature is particularly not restricted, and for example, the temperature is preferably - 80°C or higher and more preferably -20°C or higher or -15°C or higher.

When the target compound is a carbamoyl halide compound with using a base, 1 or more bases selected from a heterocyclic aromatic amine and a non-nucleophilic strong base exemplified in the above-described production condition of an isocyanate compound are preferably used. When the target compound is a urea compound, the molar ratio of the secondary amine compound to the methane or the produced carbonyl halide is preferably adjusted to more than 1. The molar ratio is preferably 1.5 or more and more preferably 2 or more.

### 7. Downstream reaction step - Production of NCA

An amino acid N-carboxy anhydride (VIII) (NCA) can be produced by using an amino acid compound (VII) in place of the alcohol compound in the above-described method for producing a carbonate compound. 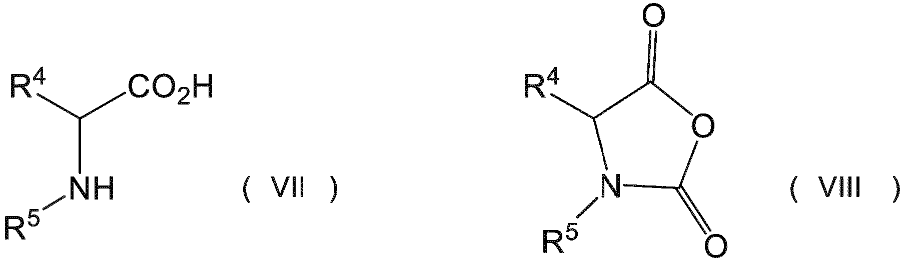 wherein
R⁴ is an amino acid side chain group wherein a reactive group is protected,
R⁵ is H or P¹-[-NH-CHR⁶-C(=O)-]ₗ- wherein R⁶ is an amino acid side chain group wherein a reactive group is protected, P¹ is a protective group of the amino group, l is an integer of 1 or more, and when l is integer of 2 or more, a plurality of R⁶ may be the same as or different from each other.

### 8. Downstream reaction step - Production of Vilsmeier reagent

A Vilsmeier reagent (X) can be produced by reacting the carbonyl halide and the amide compound (IX). The Vilsmeier reagent can be produced similarly to the above-described method for producing a carbonate compound except that the amide compound (IX) is used in place of the alcohol compound without using a base. 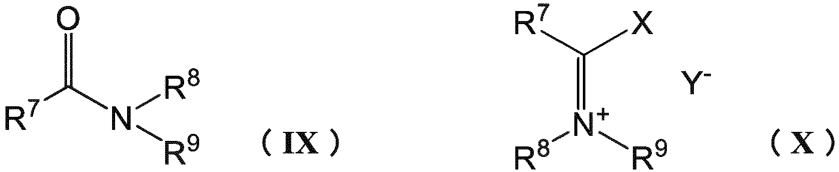 wherein
R⁷ is a hydrogen atom, a C₁₋₆ alkyl group or a C₆₋₁₂ aromatic hydrocarbon group optionally having a substituent group,
R⁸ and R⁹ are independently a C₁₋₆ alkyl group or a C₆₋₁₂ aromatic hydrocarbon group optionally having a substituent group, or R⁸ and R⁹ may form a 4 or more-membered and 7 or less-membered ring structure together with each other,
X is a halogeno group selected from the group consisting of chloro, bromo and iodo,
Y⁻ is a counter anion.

The substituent group that the C₆₋₁₂ aromatic hydrocarbon group may optionally have is particularly not restricted as long as the substituent group does not inhibit the reaction of the present invention, and an example thereof includes 1 or more substituent groups selected from the group consisting of a C₁₋₆ alkyl group, a C₁₋₆ alkoxy group, a halogeno group, a nitro group and a cyano group. The number of the substituent group is particularly not restricted as long as the C₆₋₁₂ aromatic hydrocarbon group may be substituted and may be adjusted to, for example, 1 or more and 5 or less, preferably 3 or less, more preferably 2 or less, and even more preferably 1. When the number of the substituent group is 2 or more, the substituent groups may be same as or different from each other.

An example of the 4 or more-membered and 7 or less-membered ring structure formed by R⁸, R⁹ and the nitrogen atom together with each other includes a pyrrolidyl group, a piperidyl group and a morpholino group.

An example of the specific amide compound (IX) includes N,N-dimethylformamide (DMF), N,N-dimethylacetamide (DMA), N-methyl-N-phenylformamide, N-methylpyrrolidone (NMP), 1,3-dimethylimidazolidinone (DMI), tetramethylurea, tetraethylurea and tetrabutylurea. DMF is preferred in terms of versatility and a cost.

The Y⁻ in the formula (X) is particularly not restricted, and an example of the Y⁻ in the formula (X) includes a chloride ion, a bromide ion and an iodide ion.

The usage amount of the amide compound may be appropriately adjusted as long as the reaction successfully proceeds, and the amount to 1 mL of the halomethane may be adjusted to, for example, 0.1 mol or more and 100 mol or less.

When the carbonyl halide and the amide compound are reacted, a solvent may be used. For example, the solvent may be added to the composition containing the amide compound. An example of the solvent includes a ketone solvent such as acetone, methyl ethyl ketone, methyl isobutyl ketone and cyclohexanone; an ester solvent such as ethyl acetate; an aliphatic hydrocarbon solvent such as n-hexane; an aromatic hydrocarbon solvent such as benzene, toluene, xylene and benzonitrile; an ether solvent such as diethyl ether, tetrahydrofuran and dioxane; and a nitrile solvent such as acetonitrile.

The temperature for the reaction of the carbony halide and the amide compound is particularly not restricted and may be appropriately adjusted. For example, the temperature may be adjusted to 0°C or higher and 120°C or lower. The temperature is more preferably 10°C or higher, even more preferably 20°C or higher, and more preferably 100°C or lower, even more preferably 80°C or lower or 50°C or lower.

The time to react the carbony halide and the amide compound is particularly not restricted and may be appropriately adjusted. For example, the time is preferably 0.5 hours or more and 50 hours or less. The reaction time is more preferably 1 hour or more, even more preferably 5 hours or more, and more preferably 30 hours or less, even more preferably 20 hours or less. For example, even after the production of the carbony halide is finished, the reaction mixture may be continuously stirred until the consumption of the amide compound is confirmed.

The Vilsmeier-Haack reaction using a Vilsmeier reagent allows the formylation or ketonization of an aromatic compound having an active group. In addition, it has been known that the carboxy group of a carboxylic acid compound can be transformed to a haloformyl group by a Vilsmeier reagent. Furthermore, a formate ester can be produced by reacting a Vilsmeier reagent and a hydroxy group-containing compound.

An aromatic compound having an active group means an aromatic compound activated by a substituent group or the like. The aromatic compound is hereinafter described as an "active aromatic compound". For example, a hydroxy group and an amino group such as an alkylamino group substituted by an alkyl group strongly activate an aromatic compound. In addition, an alkylcarbonylamino group (-N(C=O)R), an alkylcarbonyloxy group (-O(C=O)R), an ether group (-OR), an alkyl group (-R) (R is an alkyl group and is preferably a C₁₋₆ alkyl group) and an aromatic group activate an aromatic compound. The substituent groups are hereinafter referred to as an activating group. In addition, a compound that is constructed by condensing aromatic rings and of which conjugated system is extended, such as anthracene, is also activated and can be subjected to formylation or ketonization by a Vilsmeier reagent. The pi electron at the activated part may be electrophilically reacted with a Vilsmeier reagent to be subjected to formylation or ketonization.

The active aromatic compound is particularly not restricted as long as the active aromatic compound is activated and can be subjected to formylation or ketonization by a Vilsmeier reagent. An example of the active aromatic compound includes a C₆₋₁₀ aromatic hydrocarbon, such as benzene and naphthalene, that is substituted with the above-described activating group; a condensed aromatic hydrocarbon, such as phenanthrene and anthracene, that may be substituted with the above-described activating group; a 5-membered heteroaryl, such as pyrrol, imidazole, pyrazole, thiophen, furan, oxazole, isoxazole, thiazole, isothiazole and thiadiazole, that may be substituted with the above-described activating group; a 6-membered heteroaryl, such as pyridine, pyrazine, pyrimidine and pyridazine, that may be substituted with the above-described activating group; a condensed heteroaryl, such as indole, isoindole, quinoline, isoquinoline, benzofuran, isobenzofuran and chromene, that may be substituted with the above-described activating group. Though it has not been reported that unsubstituted furan and thiophen are subjected to formylation or ketonization by conventional Vilsmeier-Haack reaction, the formylation or ketonization at the carbon adjacent to a hetero element can be subjected to formylation or ketonization by the present invention method.

An activating group-containing aromatic compound, a carboxylic acid compound and a hydroxy group-containing compound as a substrate compound of the above-described reaction may be added to the reaction mixture after the carbony halide-containing gas is blown into the composition containing the amide compound, or added to the reaction mixture before or while the carbony halide-containing gas is blown into the composition containing the amide compound.

The usage amount of the activating group-containing aromatic compound, the carboxylic acid compound and the hydroxy group-containing compound may be appropriately adjusted, and may be adjusted to, for example, 0.1 times or more by mole and 1.0 time or less by mole to the amide compound.

A Vilsmeier reagent is also useful for producing a carboxylic acid halide from a carboxylic acid compound. A Vilsmeier reagent becomes an amide compound after the Vilsmeier reagent halogenates a carboxylic acid compound. An ester compound can be produced by reacting the produced carboxylic acid halide with an alcohol compound, and a carboxylic anhydride can be produced by reacting the produced carboxylic acid halide with a carboxylic acid. When a carboxylic acid compound and a base are used in place of an amide compound, the carboxylic acid compound may be anionized by the base and then the anionized carboxylate compound may be directly transformed to a carboxylic acid halide by the carbony halide. Such a carboxylic acid halide can be also used for producing an ester compound and a carboxylic anhydride.

### 9. Post-treatment step

Since many carbony halides are toxic, the produced carbony halide is preferably not leaked out of the reaction system. For example, it is preferred that the gas phase discharged from the reaction vessel for the reaction of the produced carbony halide is supplied into an alcohol trap, and the gas phase discharged from the alcohol trap is further supplied into an alkali trap as demonstrated in Figures 1 to 4. The alcohol trap may be cooled as long as the alcohol to be used is not coagulated. For example, the alcohol trap may be cooled to -80°C or higher and 50°C or lower. In addition, for example, a sodium hydroxide aqueous solution and a saturated sodium hydrogencarbonate aqueous solution may be used for the alkali trap.

When the compound produced from the carbony halide is a relatively unstable compound such as an isocyanate compound, a reactive substrate compound may be further added to the reaction mixture after the carbonyl halide is reacted. When the compound produced from the carbony halide is a relatively stable compound such as a carbonate compound, the target compound may be purified from the reaction mixture. For example, water and a water-insoluble organic solvent such as chloroform are added to the reaction mixture, the aqueous phase and the organic phase are separated, the organic phase is dried over anhydrous sodium sulfate or anhydrous magnesium sulfate and then concentrated under reduced pressure, and the target compound may be further purified by chromatography or the like.

The present application claims the benefit of the priority date of Japanese patent application No. 2022-203823 filed on December 21, 2022. All of the contents of the Japanese patent application No. 2022-203823 filed on December 21, 2022, are incorporated by reference herein.

### EXAMPLES

Hereinafter, the present invention is described in more detail with Examples. The present invention is however not restricted to the following Examples in any way, and it is possible to work the present invention according to the Examples with an additional appropriate change within the range of the above descriptions and the following descriptions. Such a changed embodiment is also included in the technical scope of the present invention.

### Example 1: Production of carbonyl chloride

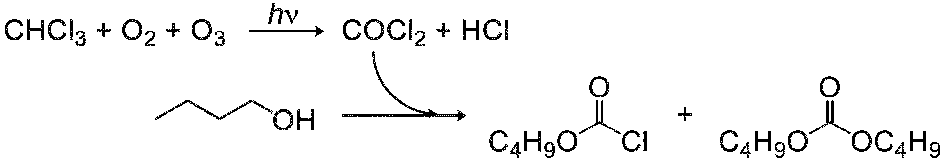

The chloroform containing 10 ppm amylene as a stabilizing agent was subjected to photooxidation with using ozone and the flow photoreaction system schematically demonstrated in Figure 1.

The chloroform was supplied from a syringe pump, joined together with a mixed gas of ozone·oxygen produced by an ozone gas generator ("PZH-05N" manufactured by KOFLOC) to be mixed at the various rates in a PTFE tube, and passed through a coil heater heated at 120°C to prepare a mixed gas of chloroform· ozone·oxygen. The mixed gas was supplied into a flow photoreaction reactor. The flow photoreaction reactor having a volume of 700 mL and a length of 180 mm was composed of a cylindrical borosilicate glass vessel (Φ 80 mm), a quartz glass inner pipe (Φ 30 mm), a 20 W low pressure mercury lamp ("SUL-20P" manufactured by SEN Light, emission part length: 130 mm) and a hot plate ("HTP452AB" manufactured by ADVANTEC) heated at 100°C. The inner pipe was built into the cylindrical borosilicate glass vessel, the low pressure mercury lamp was inserted into the inner pipe, and the hot plate was placed at the bottom part. The wavelength range of the lamp light was 185 to 600 nm, and the peak wavelength was 184.9 nm and 253.7 nm. The illumination intensity of the light having the wavelength of 185 nm at the position 5 mm away from the lamp was 2.00 to 2.81 mW/cm², and the illumination intensity of the light having the wavelength of 254 nm at the same position was 5.60 to 8.09 mW/cm². The flow rate of chloroform and ozone gas were adjusted as Table 1, and the retention time of the reaction gas in the flow photoreaction reactor was adjusted to 3.4 minutes to consider the condition of ozone photooxidation reaction of chloroform. The gas product produced from the system was blown into 1-butanol in the first reaction vessel to produce a mixed product of a chloroformate ester and a carbonate. The gas derived from the first reaction vessel was similarly supplied into 1-butanol in the second reaction vessel. The amounts of the produced ethyl chloroformate, dibutyl carbonate and carbonyl chloride were determined by analyzing the reaction mixtures in the first reaction vessel and the second reaction vessel. The result is shown in Table 1. The amount of the produced carbonyl chloride was calculated on the assumption that all of the produced carbonyl chloride were reacted with 1-butanol.

**Table 1**

| CHCl₃ | | O₂ + O₃ | | Reaction vessel | Chloroformate Bu ester | Dibutyl carbonate | Unreacted CHCl₃ | COCl₂ | Conversion efficiency vs CHCl₃ | Unreacted CHCl₃ |
|---|---|---|---|---|---|---|---|---|---|---|
| Supply rate | Supply amount | O₃ ratio | Supply rate | | | | | | | |
| [Liquid] 201.2µL/min | 299mmol | 16% | 100mL/min | First | 268mmol | 3.6mmol | 25mmol | 271mmol | 91% | 9% |
| [Gas] 77mL/min | | | | | | | | | | |
| 2.5mmol/min | | | 3.7mmol/min | Second | 1.7mmol | 0 | 0.6mmol | 1.7mmol | | |

### Example 2: Production of carbonyl chloride

The chloroform containing 10 ppm amylene as a stabilizing agent was subjected to photooxidation with using the flow photoreaction system schematically demonstrated in Figure 2.

The chloroform was supplied from a syringe pump, joined together with a mixed gas of ozone·oxygen produced by an ozone gas generator ("PZH-05N" manufactured by KOFLOC) to be mixed in a PTFE tube, and passed through a coil heater heated at 120°C to prepare a mixed gas of chloroform·ozone·oxygen. The mixed gas was supplied into a flow photoreaction reactor. The flow photoreaction reactor was composed of a borosilicate glass vessel (outer size: 250 mm × 400 mm × 50 mm, thickness: 3.3 mm, inner volume: 4157cm³), LED lamp manufactured by Polarstar (peak wavelength: 365 nm, 30 W, LED (14 cm × 18 cm)×2) and a hot plate ("HTP452AB" manufactured by ADVANTEC) heated at 100°C. The illumination intensity of the light at the position 5 mm away from the lamp was 54.7 to 54.4 mW/cm². The retention time of the reaction gas in the flow photoreaction reactor was adjusted to 35 minutes by controlling the flow rate of chloroform and ozone gas to consider the condition of ozone photooxidation reaction of chloroform. The gas product produced from the system was blown into 1-butanol in the first reaction vessel to produce a mixed product of a chloroformate ester and a carbonate. The gas derived from the first reaction vessel was similarly supplied into 1-butanol in the second reaction vessel. The amounts of the produced ethyl chloroformate, dibutyl carbonate and carbonyl chloride were determined by analyzing the reaction mixtures in the first reaction vessel and the second reaction vessel.

In addition, a similar experiment was conducted except that ozone was not supplied for comparison. The result is shown in Table 2.

**Table 2**

| CHCl₃ | | O₂ + O₃ | | COCl₂ | Conversion efficiency vs CHCl₃ | Unreacted CHCl₃ |
|---|---|---|---|---|---|---|
| Supply rate | Stabilizing agent | O₃ ratio | Supply rate | | | |
| 2.5mmol/min | amylene 10ppm | not used | 33.6mL/min | 0.075mmol/h | <0.5% | >99% |
| | amylene 10ppm | 8% | | 142mmol/h | 95% | 4% |

The chloroform containing amylene (2-pentene) as a stabilizing agent was hardly photooxidized by irradiating visible light (365 nm) as the result shown in Table 2.

On the one hand, even chloroform containing amylene as a stabilizing agent was photooxidized to produce carbonyl chloride with high yield of 95% by using ozone gas in addition to oxygen.

It was experimentally ascertained by ¹H NMR that amylene (2-pentene) was decomposed by the reaction with ozone to produce acetaldehyde and propionaldehyde.

### Example 3: Production of carbonyl chloride

Chloroform was subjected to photooxidation with using the flow photoreaction system schematically demonstrated in Figure 3. The chloroform containing amylene as a stabilizing agent, and the chloroform from which the stabilizing agent was removed and the chloroform treated with ozone to decompose amylene were used.

The chloroform was supplied from a syringe pump, joined together with a mixed gas of ozone·oxygen produced by an ozone gas generator ("PZH-05N" manufactured by KOFLOC) to be mixed in a PTFE tube, and passed through a coil heater heated at 120°C to prepare a mixed gas of chloroform·ozone·oxygen. The mixed gas was supplied into a flow photoreaction reactor. The flow photoreaction reactor was composed of a PFA tube (inner diameter: 0.2 cm, outer diameter: 0.3 cm, length: 1940 cm, volume: 61.0 cm³), LED lamp manufactured by Polarstar (peak wavelength: 365 nm, 30 W, LED (14 cm × 18 cm)×2) and a hot plate ("HTP452AB" manufactured by ADVANTEC) heated at 100°C. The illumination intensity of the light at the position 5 mm away from the lamp was 54.7 to 54.4 mW/cm². The retention time of the reaction gas in the flow photoreaction reactor was adjusted to 1.5 minutes by controlling the flow rate of chloroform and ozone gas to consider the condition of ozone photooxidation reaction of chloroform. The gas product produced from the system was blown into 1-butanol in the first reaction vessel to produce a mixed product of a chloroformate ester and a carbonate. The gas derived from the first reaction vessel was similarly supplied into 1-butanol in the second reaction vessel. The amounts of the produced ethyl chloroformate, dibutyl carbonate and carbonyl chloride were determined by analyzing the reaction mixtures in the first reaction vessel and the second reaction vessel.

In addition, a similar experiment was conducted except that an LED lamp having a peak wavelength of 405 nm was used or a light was not irradiated for comparison. The illumination intensity of the light at the position 5 mm away from the lamp was 31.6 to 32.4 mW/cm².

The result is shown in Table 3.

**Table 3**

| CHCl₃ | | O₂ + O₃ | | Irradiation wavelength | COCl₂ | Conversion efficiency vs CHCl₃ | Unreacted CHCl₃ |
|---|---|---|---|---|---|---|---|
| Supply rate | Stabilizing agent | O₃ | Supply rate | | | | |
| 0.63mmol/min | amylene | - | 16.8mL/min | 365nm | N.D. | 0% | >99.9% |
| | - | - | | 365nm | 20.8mmol/h | 27% | 71% |
| | amylene O₃ pretreatment | - | | 365nm | 8.2mmol/h | 22% | 78% |
| | - | 16% | | - | 0.8mmol/h | 1% | 98% |
| | - | 8% | | 365nm | 51.4mmol/h | 69% | 30% |
| | - | 16% | | 365nm | 56.7mmol/h | 76% | 24% |
| | - | 16% | | 405nm | 26.3mmol/h | 36% | 63% |
| | amylene | 16% | | 365nm | 53.3mmol/h | 72% | 28% |

The chloroform that contained amylene (2-pentene) as a stabilizing agent could not be photo-oxidized by oxygen and visible light (365 nm) without using ozone as the result shown in Table 3.

Though the chloroform that did not contain a stabilizing agent or the chloroform in which a stabilizing agent was decomposed by the pretreatment with using ozone was slightly photo-decomposed by irradiating visible light only, the conversion efficiency was not sufficient.

In addition, the chloroform containing a stabilizing agent was hardly oxidatively decomposed without irradiating light.

On the one hand, when ozone was added to oxygen, the yield was significantly improved. When the ratio of ozone was larger, the yield was further improved. A carbonyl chloride could be successfully produced by using ozone in combination even from the chloroform containing a stabilizing agent.

When an LED lamp of 405 nm, which was longer wavelength, was used as an irradiated light, the yield was slightly decreased. The reason why the conversion efficiency was relatively lower in such a case than those of the other Examples may be that the retention time of the gas in the reactor was short as 1.5 minutes.

### Example 4: Production of carbonyl chloride

The chloroform containing about 10 ppm amylene as a stabilizing agent was subjected to photooxidation with using ozone and the flow photoreaction system schematically demonstrated in Figure 4.

The liquid chloroform was added into a 1 L two-necked round-bottom flask to be heated and stirred at 60°C. A mixed gas of ozone·oxygen produced by an ozone gas generator ("PZH-05N" manufactured by KOFLOC) was blown into the liquid chloroform through a PTFE tube having an inner diameter of 0.2 cm and outer diameter of 0.3 cm. Light was irradiated to the flask. The flow photoreactor was composed of the round-bottom flask equipped with a condenser for cooling (0°C), a magnetic stirrer, an LED lamp (manufactured by Polarstar, 365 nm, 30 W, one LED (14 cm × 18 cm) × 1, and an aluminum block bath. The retention time of the gas in the reactor was controlled as described in Table 4 by adjusting the flow rate of the ozone gas for considering the conditions of ozone photo oxidative reaction of chloroform. The gas product produced by the system was blown into 1-butanol in a two-necked round-bottom flask to produce a mixed product of a chloroformate ester and a carbonate. The mixed product was analyzed to determine the amounts of the produced ethyl chloroformate, dibutyl carbonate and carbonyl chloride. The result is shown in Table 4. The result shown in Table 4 is the average value calculated on the basis of the result of the reaction for 2 hours.

**Table 4**

| CHCl₃ | | O₂ + O₃ | | Gas retention time | COCl₂ | Conversion efficiency vs CHCl₃ | Oxygen consumption rate vs O₂ + O₃ |
|---|---|---|---|---|---|---|---|
| Stabilizing agent | Amount | O₃ | Supply rate | | | | |
| 10ppm amylene | 80mL | - | 67.2mL/min | 14min | 19mmol/h | 2% | 6.4% |
| 10ppm amylene | 80mL | 16% | 67.2mL/min | 14min | 289mmol/h | 29% | 89% |
| 10ppm amylene | 160mL | 16% | 134mL/min | 7min | 629mmol/h | 31% | 96% |

As the result shown in Table 4, when only oxygen was supplied to the reaction system without supplying ozone, carbonyl chloride could not be sufficiently obtained, but when ozone was supplied in addition to oxygen, the yield amount of carbonyl chloride could be significantly improved.

### Example 5: Production of diphenyl carbonate

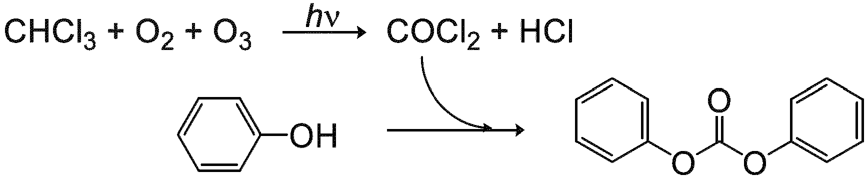

A mixed gas of ozone : oxygen = 16 vol% : 84 vol% was supplied to the photoreaction system schematically demonstrated in Figure 3 at the flow rate of 16.8 mL/min. Separately, the chloroform containing amylene as a stabilizing agent was injected at the flow rate of 50.3 µL/min to be vaporized by a heater and mixed with a mixed gas of ozone· oxygen to prepare a mixed gas of ozone·oxygen·chloroform. The mixed gas of ozone·oxygen·chloroform was continuously supplied to the photoreaction device, and light having a peak wavelength of 365 nm was irradiated thereto at 100°C. Separately, phenol (9.411 g, 100 mmol) and pyridine (12.07 mL, 150 mmol) were dissolved in dichloromethane (64 mL), and the solution was added into a two-necked flask. The gas produced by the flow photoreaction device was blown into the stirred solution at room temperature for 2 hours. To the reaction mixture, 2 M hydrochloric acid was added. The organic phase and the aqueous phase were separated, and the organic phase was washed with distilled water and dried over anhydrous sodium sulfate. The solvent was distilled away from the organic phase under reduced pressure using an evaporator, and the obtained residue was dried in vacuo at 80°C for 3 hours to obtain the target compound as a white solid (9.57 g, 44.7 mmol). The isolated yield to the raw material phenol was 89%.

Though ozone may possibly oxidize phenol to be turned brown, such coloration was not observed by the present reaction. The diphenyl carbonate produced with using ozone was less colored than the diphenyl carbonate produced similarly produced except that oxygen only was used according to visual observation. Though the mechanism is unclear, the result may be obtained by the depigmentation effect of ozone.

### Example 6: Production of polycarbonate

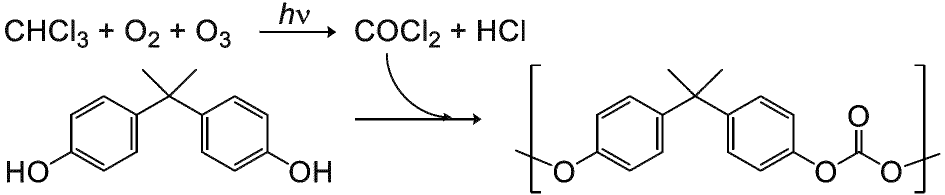

A mixed gas of ozone : oxygen = 16 vol% : 84 vol% was supplied to the photoreaction system schematically demonstrated in Figure 3 at the flow rate of 16.8 mL/min. Separately, the chloroform containing amylene as a stabilizing agent was injected at the flow rate of 50.3 µL/min to be vaporized by a heater and mixed with a mixed gas of ozone· oxygen to prepare a mixed gas of ozone·oxygen·chloroform. The mixed gas of ozone·oxygen·chloroform was continuously supplied to the flow photoreaction device, and light having a peak wavelength of 365 nm was irradiated thereto at 100°C. Separately, Bisphenol A (11.41 g, 50 mmol) and pyridine (20.12 mL, 250 mmol) were dissolved in dichloromethane (128 mL), and the solution was added into a two-necked flask. The gas produced by the flow photoreaction device was blown into the stirred solution at room temperature for 2 hours. The solution was subsequently further stirred at room temperature for 2 hours. To the reaction mixture, 2 M hydrochloric acid was added. The organic phase and the aqueous phase were separated, and the organic phase was washed with distilled water and dried over anhydrous sodium sulfate. The solvent was distilled away from the organic phase under reduced pressure using an evaporator, and n-hexane was added thereto. The produced precipitate was obtained by filtration and dried in vacuo at 80°C for 2 hours to obtain the target compound as a white solid (9.18 g, 36.1 mmol). The isolated yield to the raw material Bisphenol A was 72%.

The soluble part of the obtained polycarbonate was analyzed by GPC: gel permeation chromatography, and the molecular weight was determined in standard polystyrene equivalent. The used GPC device, column and measurement condition are hereinafter described.
Device: "LC-2000" manufactured by JASCO
Pump: "Quaternary low pressure gradient pump PU-2089" manufactured by JASCO
Detector: "Refractive Index Detector RI-4030" manufactured by JASCO
Guard column: one "TSKgel GuardcolumnH_{HR}-H" manufactured by Tosoh
Analysis column: one "TSKgelG5000H_{HR}" + one "TSKgelG4000H_{HR}" manufactured by Tosoh
Solvent: HPLC grade THF
Sample pretreatment: ADVANTEC DISMIC-03JP (pore diameter: 0.5 µm)
Amount of injected sample: 25 µL
Sample concentration: 0.2 w/v% in HPLC grade THF solution
Solvent flow rate: 1.0 mL/min
Measurement temperature: 40°C
Standard polystyrene: "Polystyrene, analytical standard, for GPC" manufactured by Chemco Plus Scientific

The result is shown as follows.

**Table 5**

| M_{w} | Mₙ | M_{w}/Mₙ |
|---|---|---|
| 306,500 | 133,300 | 2.30 |

Though ozone may possibly oxidize phenol to be turned brown, such coloration was not observed by the present reaction. The polycarbonate produced with using ozone was less colored than the polycarbonate produced similarly produced except that oxygen only was used according to visual observation. Thus, a polycarbonate having improved transparency may be produced by the present invention method.

In addition, the most part of the polycarbonate produced by the present invention method is insoluble in an organic solvent, and even the soluble part has a molecular weight that is significantly larger than that of the polycarbonate similarly produced except that only oxygen is used. Thus, not only oxidative photolytic decomposition reaction of chloroform but also polymerization reaction may be accelerated by using ozone in addition to oxygen.

### Example 7: Production of isocyanate

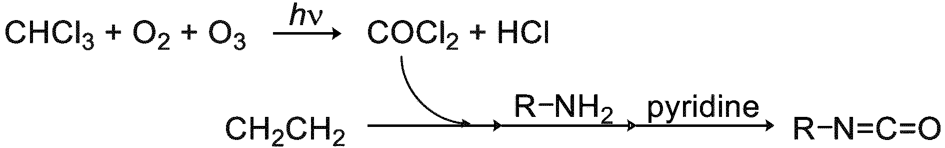

A mixed gas of ozone : oxygen = 16 vol% : 84 vol% was supplied to the photoreaction system schematically demonstrated in Figure 3 at the flow rate of 16.8 mL/min. Separately, the chloroform containing amylene as a stabilizing agent was injected at the flow rate of 50.3 µL/min to be vaporized by a heater and mixed with a mixed gas of ozone· oxygen to prepare a mixed gas of ozone·oxygen·chloroform. The mixed gas of ozone·oxygen·chloroform was continuously supplied to the flow photoreaction device, and light having a peak wavelength of 365 nm was irradiated thereto at 100°C. Separately, dichloromethane (100 mL) was added into a two-necked flask, and the gas obtained from the flow photoreaction device was blown into the stirred dichloromethane at 0°C for 1 hour or 2 hours.

The LED lamp was turned off, and the injection of the chloroform was stopped. The dichloromethane solution of the amine or diamine shown in Table 6 was injected into the above-described dichloromethane solution under a general interior light. Then, a 5-times amount by mole of pyridine to the amino group of the added amine or diamine was added thereto, and the mixture was stirred at 0°C for 1 hour.

To the reaction mixture, 1,2-dichloroethane was added as an internal standard. The mixture was analyzed by ¹H NMR; as a result, it was confirmed that an isocyanate as the target compound was produced with the yield of 96% or more. The result is shown in Table 6. The conversion efficiency in Table 6 is a conversion efficiency to the used raw material amine or raw material diamine.

**Table 6**

| Entry | Amine or Diamine | | Total amount of injected CHCl | Time for injecting gas | Pyridine | Produced isocyanate | Conversion efficiency |
|---|---|---|---|---|---|---|---|
| | Compound name | Amount | | | | | |
| 1 | Aniline | 20mmol | 38.3mmol | 1h | 100mmol | 19.3mmol | 97% |
| 2 | 1H,1H-Heptafluoro butylamine | 20mmol | 37.4mmol | 1h | 100mmol | 9.6mmol | 96% |
| 3 | 4,4'-Diaminodiphenyl methane | 10mmol | 36.3mmol | 1h | 100mmol | 9.7mmol | 97% |
| 4 | 1,6-Diamino hexane | 20mmol | 75.6mmol | 2h | 200mmol | 19.3mmol | 96% |

The reaction mixture of a similar reaction with using oxygen only is turned brown, since pyridine causes coloration. On the one hand, when ozone was used in addition to oxygen, coloration was not caused and the reaction mixture containing the product was colorless and transparent. Some factor causing coloration may be removed by ozone in the present invention method.

### Example 8: Production of carbamoyl chloride

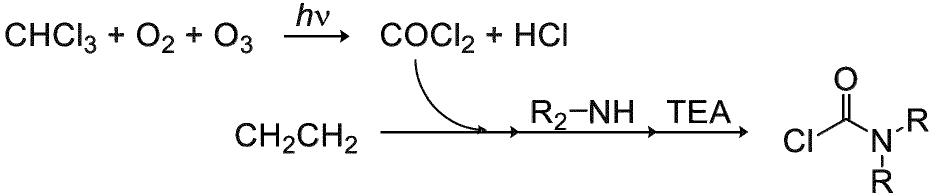

A mixed gas of ozone : oxygen = 16 vol% : 84 vol% was supplied to the photoreaction system schematically demonstrated in Figure 3 at the flow rate of 16.8 mL/min. Separately, the chloroform containing amylene as a stabilizing agent was injected at the flow rate of 26.82 µL/min to be vaporized by a heater and mixed with a mixed gas of ozone-oxygen to prepare a mixed gas of ozone-oxygen-chloroform.

The mixed gas of ozone-oxygen-chloroform was continuously supplied to the flow photoreaction device, and light having a peak wavelength of 365 nm was irradiated thereto at 100°C. Separately, dichloromethane (200 mL) was added into a two-necked flask, and the gas obtained from the flow photoreaction device was blown into the stirred dichloromethane at 0°C for 1 hour.

The LED lamp was turned off, and the injection of chloroform was stopped. The secondary amine or a salt thereof (15 mmol) shown in Table 7 was injected into the above-described dichloromethane under a general interior light. Then, a 4-times amount by mole of triethylamine (TEA) (60 mmol) to the added amine was added thereto, and the mixture was stirred at 0°C for 1 hour.

To the reaction mixture, 1,2-dichloroethane was added as an internal standard. The mixture was analyzed by ¹H NMR; as a result, it was confirmed that carbamoyl chloride as the target compound was produced with the yield of 75 to 97%. The result is shown in Table 7. The conversion efficiency in Table 7 is a conversion efficiency to the used raw material secondary amine.

**Table 7**

| Secondary amine | | | CHCl₃ | | TEA | Yield amount | Conversion efficiency (to amine) |
|---|---|---|---|---|---|---|---|
| R | Salt | Amount | Time for injection | Total injection amount | | | |
| Me | HCl | 15mmol | 1h | 20.0mmol | 60mmol | 14.6mmol | 97% |
| iPr | - | 15mmol | 1h | 20.3mmol | 60mmol | 14.2mmol | 95% |
| Et | - | 15mmol | 1h | 20.0mmol | 60mmol | 14.6mmol | 97% |
| Ph | - | 15mmol | 1h | 20.3mmol | 60mmol | 11.2mmol | 75% |

In addition, a similar experiment without using ozone and with using oxygen only was carried out for comparison. As a result, TEA caused coloration and the reaction mixture was turned brown.

On the one hand, when ozone was used in addition to oxygen, coloration was not caused and the reaction mixture containing the product was colorless and transparent. Some factor causing coloration may be removed by ozone in the present invention method.

### Example 9: Production of phenylalanine-N-carboxylic anhydride

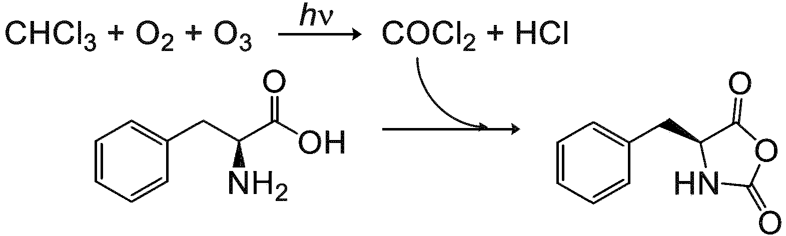

A mixed gas of ozone : oxygen = 16 : 84 by volume was supplied to the photoreaction system schematically demonstrated in Figure 3 at the flow rate of 16.8 mL/min. Separately, the chloroform containing amylene as a stabilizing agent was injected at the flow rate of 50.3 µL/min to be vaporized by a heater and mixed with a mixed gas of ozone-oxygen to prepare a mixed gas of ozone-oxygen-chloroform.

The mixed gas of ozone-oxygen-chloroform was continuously supplied to the flow photoreaction device, and light having a peak wavelength of 365 nm was irradiated thereto at 100°C. Separately, L-phenylalanine (6.6 g, 40 mmol) was dispersed in a mixed solvent of chloroform (60 mL, 740 mmol) and acetonitrile (60 mL, 1.1 mol), and the mixture was added into a two-necked flask. The gas obtained from the flow photoreaction device was blown into the stirred mixture at 70°C for 2 hours.

Then, dichloromethane (2.56 mL, 40 mmol) was added to the reaction mixture as an internal standard. The mixture was analyzed by ¹H NMR; as a result, it was confirmed that phenylalanine-N-carboxylic anhydride as the target compound was produced (yield: >99%).

### Example 10: Production of Vilsmeier reagent

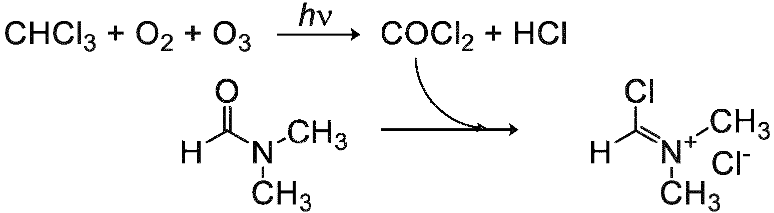

A mixed gas of ozone : oxygen = 16 : 84 by volume was supplied to the photoreaction system schematically demonstrated in Figure 3 at the flow rate of 16.8 mL/min. Separately, the chloroform containing amylene as a stabilizing agent was injected at the flow rate of 50.3 µL/min to be vaporized by a heater and mixed with a mixed gas of ozone-oxygen to prepare a mixed gas of ozone-oxygen-chloroform.

The mixed gas of ozone-oxygen-chloroform was continuously supplied to the flow photoreaction device, and light having a peak wavelength of 365 nm was irradiated thereto at 100°C. Separately, DMF (1.55 mL, 20 mmol) was dissolved in chloroform (20 mL), and the solution was added into a two-necked flask. The gas obtained from the flow photoreaction device was blown into the stirred solution at room temperature for 1 hour. Subsequently, the supply of the gas and the irradiation of light were stopped, and the reaction mixture was stirred at 50°C for 1.5 hours. Then, when the stirring was stopped, the reaction mixture was separated into two layers.

Each layer was analyzed by ¹H NMR; as a result, the peak of a Vilsmeier reagent was hardly recognized in the lower layer. On the one hand, the peaks of (chloromethylene)dimethyliminium chloride as a Vilsmeier reagent was recognized in the upper layer. The ratio of the remaining DMF and a Vilsmeier reagent (DMF : Vilsmeier reagent) in the upper layer was about 1:6 in accordance with the peak intensities, and it was estimated that up to 17 mmol of a Vilsmeier reagent was produced.

## Claims

1. A method for producing a carbonyl halide, comprising the step of irradiating a light to a halomethane having 1 or more halogeno groups selected from the group consisting of chloro, bromo and iodo in the presence of oxygen and ozone.

2. The method according to claim 1, wherein the light is irradiated to a mixed gas comprising the halomethane, the oxygen and the ozone.

3. The method according to claim 1, wherein a ratio of the ozone to a total of the oxygen and the ozone is 1 vol% or more and 20 vol% or less.

4. The method according to claim 2, wherein a volume ratio of the halomethane to the ozone is 0.1 times or more and 50 times or less.

5. The method according to claim 2, wherein the mixed gas does not comprise chlorine.

6. The method according to claim 1, wherein the light is irradiated to the halomethane for 60 seconds or more and 5000 seconds or less.

7. The method according to claim 1, wherein the light is irradiated to the halomethane at a temperature of 50°C or higher and 200°C or lower.

8. A method for producing a carbonate compound, the method comprising the steps of:
producing a carbonyl halide by the method according to any one of claims 1 to 7, and
reacting an alcohol compound and the carbonyl halide.

9. A method for producing a halogenated formate ester compound, the method comprising the steps of:
producing a carbonyl halide by the method according to any one of claims 1 to 7, and
reacting an alcohol compound and the carbonyl halide.

10. A method for producing an isocyanate compound, the method comprising the steps of:
producing a carbonyl halide by the method according to any one of claims 1 to 7, and
reacting a primary amine compound and the carbonyl halide.

11. A method for producing a carbamoyl halide compound, the method comprising the steps of:
producing a carbonyl halide by the method according to any one of claims 1 to 7, and
reacting a secondary amine compound and the carbonyl halide.

12. A method for producing an amino acid N-carboxy anhydride,
the method comprising the steps of:
producing a carbonyl halide by the method according to any one of claims 1 to 7, and
reacting an amino acid compound represented by the following formula (VII) and the carbonyl halide,
wherein the amino acid N-carboxy anhydride is represented by the following formula (VIII): 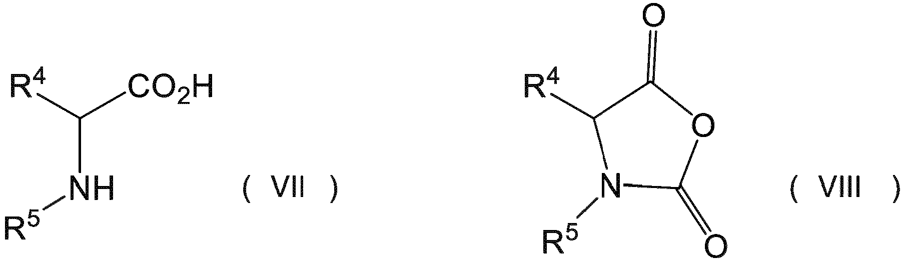 wherein
R⁴ is an amino acid side chain group wherein a reactive group of the amino acid side chain group is protected,
R⁵ is H or P¹-[-NH-CHR⁶-C(=O)-]₁- wherein R⁶ is an amino acid side chain group wherein a reactive group of the amino acid side chain group is protected, P¹ is a protective group of the amino group, l is an integer of 1 or more, a plurality of R⁶ is the same as or different from each other in the case where l is an integer of 2 or more.

13. A method for producing a Vilsmeier reagent,
wherein the Vilsmeier reagent is a salt represented by the following formula (X): 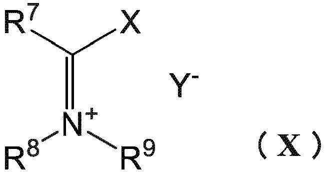 wherein
R⁷ is a hydrogen atom, a C₁₋₆ alkyl group or a C₆₋₁₂ aromatic hydrocarbon group optionally having a substituent group,
R⁸ and R⁹ are independently a C₁₋₆ alkyl group or a C₆₋₁₂ aromatic hydrocarbon group optionally having a substituent group, or R⁸ and R⁹ may form a 4 or more-membered and 7 or less-membered ring structure together with each other,
X is a halogeno group selected from the group consisting of chloro, bromo and iodo,
Y⁻ is a counter anion,
the method comprising the steps of:
producing a carbonyl halide by the method according to any one of claims 1 to 7, and
reacting the carbonyl halide and an amide compound represented by the following formula (IX): 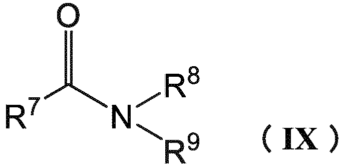 wherein R⁷ to R⁹ have the same meanings as the above.
